# EUROPEAN PATENT APPLICATION

(11) **EP 2 910 645 A1**
(43) Date of publication of application: **26.08.2015**
(21) Application number: 14156501.0
(22) Date of filing: 25.02.2014
(51) Int. Cl.: C12Q 1/68

(54) **Methods and compositions for prediction of therapeutic efficacy of cancer treatments and cancer prognosis upon antibody treatment**

(71) Applicant: STRATIFYER Molecular Pathology GmbH, 50935 Köln (DE)
(72) Inventor: Wirtz, Ralph M., 50677 Köln (DE)
(74) Representative: Féaux de Lacroix, Stefan

(57) **Abstract**

The invention generally relates to methods and compositions for the prediction of therapeutic efficacy of cancer treatments and the prognosis of cancer. The invention discloses markers that are associated with favorable and unfavorable outcomes, respectively, in certain cancer treatments and are useful as prognostic markers for cancer. Methods involving these markers are disclosed for predicting cancer therapy benefit and prognosing clinical outcome for cancer patients.

<110>  STRATIFYER Molecular Pathology GmbH et al.

<120>  METHODS AND COMPOSITIONS FOR PREDICTION OF THERAPEUTIC EFFICACY
       OF CANCER TREATMENTS AND CANCER PROGNOSIS

<130>  STR69048EP

<160>  7

<170>  PatentIn version 3.5

<210>  1
<211>  354
<212>  PRT
<213>  Homo sapiens

<400>  1

Met Arg Leu Ala Val Leu Phe Ser Gly Ala Leu Leu Gly Leu Leu Ala
1               5                   10                  15

Ala Gln Gly Thr Gly Asn Asp Cys Pro His Lys Lys Ser Ala Thr Leu
            20                  25                  30

Leu Pro Ser Phe Thr Val Thr Pro Thr Val Thr Glu Ser Thr Gly Thr
        35                  40                  45

Thr Ser His Arg Thr Thr Lys Ser His Lys Thr Thr Thr His Arg Thr
    50                  55                  60

Thr Thr Thr Gly Thr Thr Ser His Gly Pro Thr Thr Ala Thr His Asn
65                  70                  75                  80

Pro Thr Thr Thr Ser His Gly Asn Val Thr Val His Pro Thr Ser Asn
                85                  90                  95

Ser Thr Ala Thr Ser Gln Gly Pro Ser Thr Ala Thr His Ser Pro Ala
            100                 105                 110

Thr Thr Ser His Gly Asn Ala Thr Val His Pro Thr Ser Asn Ser Thr
        115                 120                 125

Ala Thr Ser Pro Gly Phe Thr Ser Ser Ala His Pro Glu Pro Pro Pro
    130                 135                 140

Pro Ser Pro Ser Pro Ser Pro Thr Ser Lys Glu Thr Ile Gly Asp Tyr
145                 150                 155                 160

Thr Trp Thr Asn Gly Ser Gln Pro Cys Val His Leu Gln Ala Gln Ile
                165                 170                 175

Gln Ile Arg Val Met Tyr Thr Thr Gln Gly Gly Gly Glu Ala Trp Gly

180                 185                 190

Ile Ser Val Leu Asn Pro Asn Lys Thr Lys Val Gln Gly Ser Cys Glu
    195                 200                 205

Gly Ala His Pro His Leu Leu Leu Ser Phe Pro Tyr Gly His Leu Ser
    210                 215                 220

Phe Gly Phe Met Gln Asp Leu Gln Gln Lys Val Val Tyr Leu Ser Tyr
225                 230                 235                 240

Met Ala Val Glu Tyr Asn Val Ser Phe Pro His Ala Ala Gln Trp Thr
                245                 250                 255

Phe Ser Ala Gln Asn Ala Ser Leu Arg Asp Leu Gln Ala Pro Leu Gly
            260                 265                 270

Gln Ser Phe Ser Cys Ser Asn Ser Ser Ile Ile Leu Ser Pro Ala Val
        275                 280                 285

His Leu Asp Leu Leu Ser Leu Arg Leu Gln Ala Ala Gln Leu Pro His
    290                 295                 300

Thr Gly Val Phe Gly Gln Ser Phe Ser Cys Pro Ser Asp Arg Ser Ile
305                 310                 315                 320

Leu Leu Pro Leu Ile Ile Gly Leu Ile Leu Leu Gly Leu Leu Ala Leu
                325                 330                 335

Val Leu Ile Ala Phe Cys Ile Ile Arg Arg Arg Pro Ser Ala Tyr Gln
            340                 345                 350

Ala Leu

<210>  2
<211>  1739
<212>  DNA
<213>  Homo sapiens

<400>  2
gggagcaggg ttgagcaact ggtgcagaca gcctagctgg actttgggtg aggcggttca      60

gccatgaggc tggctgtgct tttctcgggg gccctgctgg ggctactggc agcccagggg     120

acagggaatg actgtcctca caaaaaatca gctactttgc tgccatcctt cacggtgaca     180

cccacggtta cagagagcac tggaacaacc agccacagga ctaccaagag ccacaaaacc     240

accactcaca ggacaaccac cacaggcacc accagccacg gacccacgac tgccactcac     300

aaccccacca ccaccagcca tggaaacgtc acagttcatc caacaagcaa tagcactgcc     360

accagccagg gaccctcaac tgccactcac agtcctgcca ccactagtca tggaaatgcc 420

acggttcatc caacaagcaa cagcactgcc accagcccag gattcaccag ttctgcccac 480

ccagaaccac ctccaccctc tccgagtcct agcccaacct ccaaggagac cattggagac 540

tacacgtgga ccaatggttc ccagccctgt gtccacctcc aagcccagat tcagattcga 600

gtcatgtaca caacccaggg tggaggagag gcctggggca tctctgtact gaaccccaac 660

aaaaccaagg tccagggaag ctgtgagggt gcccatcccc acctgcttct ctcattcccc 720

tatggacacc tcagctttgg attcatgcag gacctccagc agaaggttgt ctacctgagc 780

tacatggcgg tggagtacaa tgtgtccttc ccccacgcag cacagtggac attctcggct 840

cagaatgcat cccttcgaga tctccaagca cccctggggc agagcttcag ttgcagcaac 900

tcgagcatca ttctttcacc agctgtccac ctcgacctgc tctccctgag gctccaggct 960

gctcagctgc cccacacagg ggtctttggg caaagtttct cctgccccag tgaccggtcc 1020

atcttgctgc ctctcatcat cggcctgatc cttcttggcc tcctcgccct ggtgcttatt 1080

gctttctgca tcatccggag acgcccatcc gcctaccagg ccctctgagc atttgcttca 1140

aaccccaggg cactgagggg gttggggtgt ggtggggggg taccctattt tcctcgacac 1200

gcaactggct caaagacaat gttattttcc ttccctttct tgaagaacaa aaagaaagcc 1260

gggcatgacg gctcatgcct gtaatcccag cactttggga ggctgaggca ggtggatcac 1320

tggaggtcag gagtttgaga ccagcctggc caacatggtg aaaccctgtc tctactaaaa 1380

atacaattag ccaggtgtgg cggcgtaatc ccagctggcc tgtaatccca gctacttggg 1440

aggctgaggc agaactgctt gaacccagga ggtggaggtt gcagtgagcc gtcatcgcgc 1500

cactaagcca agatcgcgcc actgcactcc agcctgggcg acagagccag actgtctcaa 1560

ataaataaat atgagataat gcagtcggga gaagggaggg agagaatttt attaaatgtg 1620

acgaactgcc cccccccccc cagcaggaga gcagcaaaat ttatgcaaat ctttgacggg 1680

gttttccttg tcctgccagg attaaaagcc atgagtttct taaaaaaaaa aaaaaaaaa 1739

<210> 3
<211> 1225
<212> PRT
<213> Homo sapiens

<400> 3

Met Lys Leu Arg Leu Pro Ala Ser Pro Glu Thr His Leu Asp Met Leu
1               5                   10                  15

Arg His Leu Tyr Gln Gly Cys Gln Val Val Gln Gly Asn Leu Glu Leu
            20                  25                  30

Thr Tyr Leu Pro Thr Asn Ala Ser Leu Ser Phe Leu Gln Asp Ile Gln
        35                  40                  45

Glu Val Gln Gly Tyr Val Leu Ile Ala His Asn Gln Val Arg Gln Val
    50                  55                  60

Pro Leu Gln Arg Leu Arg Ile Val Arg Gly Thr Gln Leu Phe Glu Asp
65                  70                  75                  80

Asn Tyr Ala Leu Ala Val Leu Asp Asn Gly Asp Pro Leu Asn Asn Thr
                85                  90                  95

Thr Pro Val Thr Gly Ala Ser Pro Gly Gly Leu Arg Glu Leu Gln Leu
            100                 105                 110

Arg Ser Leu Thr Glu Ile Leu Lys Gly Gly Val Leu Ile Gln Arg Asn
        115                 120                 125

Pro Gln Leu Cys Tyr Gln Asp Thr Ile Leu Trp Lys Asp Ile Phe His
    130                 135                 140

Lys Asn Asn Gln Leu Ala Leu Thr Leu Ile Asp Thr Asn Arg Ser Arg
145                 150                 155                 160

Ala Cys His Pro Cys Ser Pro Met Cys Lys Gly Ser Arg Cys Trp Gly
                165                 170                 175

Glu Ser Ser Glu Asp Cys Gln Ser Leu Thr Arg Thr Val Cys Ala Gly
            180                 185                 190

Gly Cys Ala Arg Cys Lys Gly Pro Leu Pro Thr Asp Cys Cys His Glu
        195                 200                 205

Gln Cys Ala Ala Gly Cys Thr Gly Pro Lys His Ser Asp Cys Leu Ala
    210                 215                 220

Cys Leu His Phe Asn His Ser Gly Ile Cys Glu Leu His Cys Pro Ala
225                 230                 235                 240

Leu Val Thr Tyr Asn Thr Asp Thr Phe Glu Ser Met Pro Asn Pro Glu
                245                 250                 255

Gly Arg Tyr Thr Phe Gly Ala Ser Cys Val Thr Ala Cys Pro Tyr Asn
            260                 265                 270

Tyr Leu Ser Thr Asp Val Gly Ser Cys Thr Leu Val Cys Pro Leu His
        275                 280                 285

Asn Gln Glu Val Thr Ala Glu Asp Gly Thr Gln Arg Cys Glu Lys Cys
    290                 295                 300

Ser Lys Pro Cys Ala Arg Val Cys Tyr Gly Leu Gly Met Glu His Leu
305                 310                 315                 320

Arg Glu Val Arg Ala Val Thr Ser Ala Asn Ile Gln Glu Phe Ala Gly
                325                 330                 335

Cys Lys Lys Ile Phe Gly Ser Leu Ala Phe Leu Pro Glu Ser Phe Asp
            340                 345                 350

Gly Asp Pro Ala Ser Asn Thr Ala Pro Leu Gln Pro Glu Gln Leu Gln
        355                 360                 365

Val Phe Glu Thr Leu Glu Glu Ile Thr Gly Tyr Leu Tyr Ile Ser Ala
    370                 375                 380

Trp Pro Asp Ser Leu Pro Asp Leu Ser Val Phe Gln Asn Leu Gln Val
385                 390                 395                 400

Ile Arg Gly Arg Ile Leu His Asn Gly Ala Tyr Ser Leu Thr Leu Gln
                405                 410                 415

Gly Leu Gly Ile Ser Trp Leu Gly Leu Arg Ser Leu Arg Glu Leu Gly
            420                 425                 430

Ser Gly Leu Ala Leu Ile His His Asn Thr His Leu Cys Phe Val His
        435                 440                 445

Thr Val Pro Trp Asp Gln Leu Phe Arg Asn Pro His Gln Ala Leu Leu
    450                 455                 460

His Thr Ala Asn Arg Pro Glu Asp Glu Cys Val Gly Glu Gly Leu Ala
465                 470                 475                 480

Cys His Gln Leu Cys Ala Arg Gly His Cys Trp Gly Pro Gly Pro Thr
                485                 490                 495

Gln Cys Val Asn Cys Ser Gln Phe Leu Arg Gly Gln Glu Cys Val Glu
            500                 505                 510

Glu Cys Arg Val Leu Gln Gly Leu Pro Arg Glu Tyr Val Asn Ala Arg
        515                 520                 525

His Cys Leu Pro Cys His Pro Glu Cys Gln Pro Gln Asn Gly Ser Val
    530                 535                 540

Thr Cys Phe Gly Pro Glu Ala Asp Gln Cys Val Ala Cys Ala His Tyr
545                 550                 555                 560

Lys Asp Pro Pro Phe Cys Val Ala Arg Cys Pro Ser Gly Val Lys Pro

                565                 570                 575

Asp Leu Ser Tyr Met Pro Ile Trp Lys Phe Pro Asp Glu Glu Gly Ala
            580                 585                 590

Cys Gln Pro Cys Pro Ile Asn Cys Thr His Ser Cys Val Asp Leu Asp
        595                 600                 605

Asp Lys Gly Cys Pro Ala Glu Gln Arg Ala Ser Pro Leu Thr Ser Ile
    610                 615                 620

Ile Ser Ala Val Val Gly Ile Leu Leu Val Val Val Leu Gly Val Val
625                 630                 635                 640

Phe Gly Ile Leu Ile Lys Arg Arg Gln Gln Lys Ile Arg Lys Tyr Thr
                645                 650                 655

Met Arg Arg Leu Leu Gln Glu Thr Glu Leu Val Glu Pro Leu Thr Pro
            660                 665                 670

Ser Gly Ala Met Pro Asn Gln Ala Gln Met Arg Ile Leu Lys Glu Thr
        675                 680                 685

Glu Leu Arg Lys Val Lys Val Leu Gly Ser Gly Ala Phe Gly Thr Val
    690                 695                 700

Tyr Lys Gly Ile Trp Ile Pro Asp Gly Glu Asn Val Lys Ile Pro Val
705                 710                 715                 720

Ala Ile Lys Val Leu Arg Glu Asn Thr Ser Pro Lys Ala Asn Lys Glu
                725                 730                 735

Ile Leu Asp Glu Ala Tyr Val Met Ala Gly Val Gly Ser Pro Tyr Val
            740                 745                 750

Ser Arg Leu Leu Gly Ile Cys Leu Thr Ser Thr Val Gln Leu Val Thr
        755                 760                 765

Gln Leu Met Pro Tyr Gly Cys Leu Leu Asp His Val Arg Glu Asn Arg
    770                 775                 780

Gly Arg Leu Gly Ser Gln Asp Leu Leu Asn Trp Cys Met Gln Ile Ala
785                 790                 795                 800

Lys Gly Met Ser Tyr Leu Glu Asp Val Arg Leu Val His Arg Asp Leu
                805                 810                 815

Ala Ala Arg Asn Val Leu Val Lys Ser Pro Asn His Val Lys Ile Thr
            820                 825                 830

Asp Phe Gly Leu Ala Arg Leu Leu Asp Ile Asp Glu Thr Glu Tyr His
        835                 840                 845

Ala Asp Gly Gly Lys Val Pro Ile Lys Trp Met Ala Leu Glu Ser Ile
    850                 855                 860

Leu Arg Arg Arg Phe Thr His Gln Ser Asp Val Trp Ser Tyr Gly Val
865                 870                 875                 880

Thr Val Trp Glu Leu Met Thr Phe Gly Ala Lys Pro Tyr Asp Gly Ile
                885                 890                 895

Pro Ala Arg Glu Ile Pro Asp Leu Leu Glu Lys Gly Glu Arg Leu Pro
            900                 905                 910

Gln Pro Pro Ile Cys Thr Ile Asp Val Tyr Met Ile Met Val Lys Cys
        915                 920                 925

Trp Met Ile Asp Ser Glu Cys Arg Pro Arg Phe Arg Glu Leu Val Ser
    930                 935                 940

Glu Phe Ser Arg Met Ala Arg Asp Pro Gln Arg Phe Val Val Ile Gln
945                 950                 955                 960

Asn Glu Asp Leu Gly Pro Ala Ser Pro Leu Asp Ser Thr Phe Tyr Arg
                965                 970                 975

Ser Leu Leu Glu Asp Asp Asp Met Gly Asp Leu Val Asp Ala Glu Glu
            980                 985                 990

Tyr Leu Val Pro Gln Gln Gly Phe Phe Cys Pro Asp Pro Ala Pro Gly
        995                 1000                1005

Ala Gly Gly Met Val His His Arg His Arg Ser Ser Ser Thr Arg
    1010                1015                1020

Ser Gly Gly Gly Asp Leu Thr Leu Gly Leu Glu Pro Ser Glu Glu
    1025                1030                1035

Glu Ala Pro Arg Ser Pro Leu Ala Pro Ser Glu Gly Ala Gly Ser
    1040                1045                1050

Asp Val Phe Asp Gly Asp Leu Gly Met Gly Ala Ala Lys Gly Leu
    1055                1060                1065

Gln Ser Leu Pro Thr His Asp Pro Ser Pro Leu Gln Arg Tyr Ser
    1070                1075                1080

Glu Asp Pro Thr Val Pro Leu Pro Ser Glu Thr Asp Gly Tyr Val
    1085                1090                1095

Ala Pro Leu Thr Cys Ser Pro Gln Pro Glu Tyr Val Asn Gln Pro
    1100                1105                1110

Asp Val Arg Pro Gln Pro Pro Ser Pro Arg Glu Gly Pro Leu Pro
    1115                1120                1125

Ala Ala Arg Pro Ala Gly Ala Thr Leu Glu Arg Pro Lys Thr Leu
    1130                1135                1140

Ser Pro Gly Lys Asn Gly Val Val Lys Asp Val Phe Ala Phe Gly
    1145                1150                1155

Gly Ala Val Glu Asn Pro Glu Tyr Leu Thr Pro Gln Gly Gly Ala
    1160                1165                1170

Ala Pro Gln Pro His Pro Pro Pro Ala Phe Ser Pro Ala Phe Asp
    1175                1180                1185

Asn Leu Tyr Tyr Trp Asp Gln Asp Pro Pro Glu Arg Gly Ala Pro
    1190                1195                1200

Pro Ser Thr Phe Lys Gly Thr Pro Thr Ala Glu Asn Pro Glu Tyr
    1205                1210                1215

Leu Gly Leu Asp Val Pro Val
    1220                1225

<210> 4
<211> 1255
<212> PRT
<213> Homo sapiens

<400> 4

<210>  5
<211>  22
<212>  DNA
<213>  Artificial Sequence

<220>
<223>  Oligonucleotide

<400>  5
cagcaactcg agcatcattc tt                                              22

<210>  6
<211>  21
<212>  DNA
<213>  Artificial Sequence

<220>
<223>  Oligonucleotide

<400>  6
gcaggagaaa ctttgcccaa a                                               21

<210>  7
<211>  25
<212>  DNA
<213>  Artificial Sequence

<220>
<223>  Oligonucleotide

<400>  7
tccacctcga cctgctctcc ctgag                                              25

## Description

### TECHNICAL FIELD OF THE INVENTION

The invention generally relates to methods and compositions for the prediction of therapeutic efficacy of cancer treatments and the prognosis of cancer. The invention discloses markers that are associated with favorable and unfavorable outcomes, respectively, in certain cancer treatments and are useful as prognostic markers for cancer. Methods involving these markers are disclosed for predicting cancer therapy benefit and prognosing clinical outcome for cancer patients.

### BACKGROUND OF THE INVENTION

Chromosomal aberrations are of importance for the development of cancer, as they account for deregulations of the respective regions. Amplification has been described for the genomic region carrying the HER2/neu gene in breast cancer patients. In approximately 25% of breast cancer patients the HER2/neu gene is overexpressed due to gene amplification. HER2 (human epidermal growth factor receptor 2) also known as Neu, ErbB-2, CD340 or p185 is a protein that in humans is encoded by the ERBB2 gene. HER2 is a member of the epidermal growth factor receptor (EGFR/ErbB) family. The ErbB family is composed of four plasma membrane-bound receptor tyrosine kinases which contain an extracellular ligand binding domain, a transmembrane domain, and an intracellular domain that can interact with a multitude of signaling molecules and exhibit both ligand-dependent and ligand-independent activity. HER2 can heterodimerise with any of the other three receptors and is considered to be the preferred dimerisation partner of the other ErbB receptors. Dimerisation results in the autophosphorylation of tyrosine residues within the cytoplasmic domain of the receptors and initiates a variety of signaling pathways.

Amplification of the HER2/neu gene has been shown to play an important role in the pathogenesis and progression of certain aggressive types of breast cancer and in recent years it has evolved to become an important biomarker and target of therapy for the disease. HER2/neu overexpression correlates with a poor prognosis. Gene specific antibodies raised against HER2/neu have been generated to treat cancers where HER2 is over-expressed. For example, HER2 is the target of the monoclonal antibody trastuzumab (marketed as HerceptinTM). There is a relative risk reduction of 50% for developing distant metastasis upon adjuvant treatment of HER2 positive patients with trastuzumab in combination with taxane treatment. However, the absolute benefit for reduction is 15% for early relapse and at about 3% overall survival. The single agent effectivity of Herceptin is lower, indicating a synergism of trastuzumab efficacy with chemotherapy. The discrepancy of HER2/neu positive tumors (overexpressing HER2/neu to similar extent) with regard to responsiveness to therapeutic intervention suggests that there are additional factors which are associated with therapy outcome.

Cancer therapies involving monoclonal antibodies such as trastuzumab are generally expensive and at least some monoclonal antibodies are associated with toxicity for the patient. For example, trastuzumab has been associated with cardiac toxicity. These disadvantages demand careful selection of patients who may have a benefit from antibody therapy. On the other side, non-targeted therapies such as traditional chemotherapy cause significant, and often dangerous, side effects due to non-specific effects on cells whether normal or malignant.

Therefore, there is a need to develop a test to measure the eligibility of patients for certain cancer therapies, in particular antibody therapy and chemotherapy, respectively. The present invention addresses this need by providing markers which are associated with favorable and unfavorable outcomes, respectively, in antibody therapy and chemotherapy for cancer. Furthermore, the present invention demonstrates that these markers are useful as markers for prognosing clinical outcome for cancer patients.

The findings presented herein may be used to select a suitable treatment for a cancer patient and, in particular, to decide whether antibody therapy, chemotherapy or both should be administered to a cancer patient.

### SUMMARY OF THE INVENTION

The present invention is based on the finding that macrophage markers are associated with sensitivity of cancer towards antibody treatment such as treatment of breast cancer with trastuzumab and sensitivity of cancer towards chemotherapy treatment such as treatment of breast cancer with taxanes or anthracyclines. The present invention is further based on the finding that macrophage markers are associated with clinical outcome for cancer patients and thus are useful for prognosing cancer. In particular, high levels of macrophage markers such as CD68 were found to be predictive for responsiveness of cancer patients to antibody treatment. Moreover, high levels of macrophage markers such as CD68 were found to be predictive for responsiveness of cancer patients to chemotherapy. Low levels of macrophage markers such as CD68 were found to be predictive for non-responsiveness of cancer patients to antibody treatment. Low levels of macrophage markers such as CD68 were found to be predictive for both, non-responsiveness of cancer patients to chemotherapy and antibody based regimen. However, low levels of macrophage markers such as CD68 were found to be generally prognostic for favorable clinical outcome of cancer patients irrespective of the applied adjuvant or neoadjuvant treatment. High levels of macrophage markers such as CD68 were found to be predictive for unfavorable clinical outcome of cancer patients, if not treated with antibody regimen.

In one aspect, the invention relates to a method of assessing if a cancer patient having a tumor antigen-positive tumor is a responder to treatment with an antibody against the tumor antigen acting through recruiting the patient's immune system to destroy tumor cells, said method comprising determining the level of cells of the macrophage lineage in a tumor sample obtained from the patient.

In one embodiment, the presence of cells of the macrophage lineage or a level of cells of the macrophage lineage which is higher than a defined threshold indicates an increased risk of recurrence but also an increased chance of being a responder to treatment with the antibody. In one embodiment, the absence of cells of the macrophage lineage or a level of cells of the macrophage lineage which is lower than a defined threshold indicates a reduced risk of a cancer patient being a responder to treatment with the antibody.

In one embodiment, the level of cells of the macrophage lineage is determined by determining the expression level of one or more of CD68, CD14, CD163, CD80, MAC2 (galectin-3), EMR1-F4/80, CD11b and CD205.

In one embodiment, the tumor antigen is the HER2 protein.

In a further aspect, the invention relates to a method of assessing if a cancer patient having a HER2-positive tumor is a responder to treatment with an antibody against the HER2 protein acting through recruiting the patient's immune system to destroy tumor cells, said method comprising determining the expression level of CD68 in a tumor sample obtained from the patient.

In one embodiment, expression of CD68 or an expression level of CD68 which is lower than a defined expression threshold indicates an increased risk of a cancer patient not being a responder to treatment with the antibody. In one embodiment, higher expression of CD68 or an expression level of CD68 which is higher than a defined expression threshold indicates a reduced risk of a cancer patient not being a responder to treatment with the antibody.

In one embodiment of the above aspects of the invention, the antibody acts through antibody-dependent cell-mediated cytotoxicity (ADCC) and/or complement-dependent cytotoxicity (CDC). In one embodiment of the above aspects of the invention, the antibody is a monoclonal antibody. In one embodiment of the above aspects of the invention, the antibody is trastuzumab (Herceptin) pertuzumab or trastuzumab emtasine (T-DM1). In one embodiment of the above aspects of the invention, non-responsiveness to treatment with the antibody comprises a relative reduction in one or more of survival, recurrence-free survival, and distant recurrence-free survival. In one embodiment of the above aspects of the invention, the patient is treated with adjuvant chemotherapy, wherein the adjuvant chemotherapy may comprise treatment with a taxane such as docetaxel or vinorelbine. In one embodiment, the adjuvant chemotherapy further comprises treatment with an anthracycline such as epirubicin and optionally one or more of fluorouracil and cyclophosphamide.

In a further aspect, the invention relates to a method of treating a cancer patient, said method comprising
a. assessing if the cancer patient is a responder to treatment with an antibody acting through recruiting the patient's immune system to destroy tumor cells by the method of the invention and
b. (i) treating the cancer patient with an antibody acting through recruiting the patient's immune system to destroy tumor cells if the patient has a reduced risk for not being a responder to treatment with the antibody or (ii) treating the cancer patient with a treatment regimen which comprises a treatment which is different from a treatment with an antibody acting through recruiting the patient's immune system to destroy tumor cells if the patient has an increased risk for not being a responder to treatment with the antibody, wherein said treatment regimen preferably does not comprise a treatment with an antibody acting through recruiting the patient's immune system to destroy tumor cells.

In one embodiment, the treatment regimen comprises a treatment not being dependent on the immune system of the patient. In one embodiment, the treatment regimen comprises surgery, chemotherapy, radiation and/or treatment with small molecules affecting receptor tyrosinekinases (such as CSF1R inhibitors). In one embodiment, the treatment regimen comprises a treatment with a small molecule inhibitor of the tumor antigen and/or an antibody-drug conjugate wherein the antibody is directed against the tumor antigen. In one embodiment, the antibody-drug conjugate is an antibody coupled to a radioactive, chemotherapeutic or toxin moiety. In one embodiment, the antibody-drug conjugate is an antibody coupled to a cytostatic or cytotoxic compound.

In a further aspect, the invention relates to a method of assessing if a cancer patient is a responder to treatment with chemotherapy, said method comprising determining the level of cells of the macrophage lineage in a tumor sample obtained from the patient.

In one embodiment, the presence of cells of the macrophage lineage or a level of cells of the macrophage lineage which is higher than a defined threshold indicates a reduced risk of a cancer patient not being a responder to treatment with chemotherapy. In one embodiment, the absence of cells of the macrophage lineage or a level of cells of the macrophage lineage which is lower than a defined threshold indicates an increased risk of a cancer patient not being a responder to treatment with chemotherapy or from benefiting from chemotherapy.

In one embodiment, the level of cells of the macrophage lineage is determined by determining the expression level of one or more macrophage specific markers, wherein the one or more macrophage specific markers preferably are selected from the group consisting of CD68, CD14, CD163, CD80, MAC2 (galectin-3), EMR1-F4/80, CD11b and CD205.

In a further aspect, the invention relates to a method of assessing if a cancer patient is a responder to treatment with chemotherapy, said method comprising determining the expression level of CD68 in a tumor sample obtained from the patient.

In one embodiment, expression of CD68 or an expression level of CD68 which is higher than a defined expression threshold indicates a reduced risk of a cancer patient not being a responder to treatment with chemotherapy or benefiting from chemotherapy. In one embodiment, no expression of CD68 or an expression level of CD68 which is lower than a defined expression threshold indicates an increased risk of a cancer patient not being a responder to treatment with chemotherapy or benefiting from chemotherapy.

In one embodiment of the above aspects of the invention, non-responsiveness to treatment with chemotherapy comprises a relative reduction in one or more of pathological complete response, survival, recurrence-free survival, and distant recurrence-free survival. In one embodiment of the above aspects of the invention, the cancer patient has a HER2-negative tumor. In one embodiment of the above aspects of the invention, the cancer patient has a estrogen receptor 1 (ESR1)-positive or estrogen receptor 1 (ESR1)-negative tumor. In one embodiment of the above aspects of the invention, the chemotherapy comprises treatment with one or more of taxanes, anthracyclines and platinum compounds.

In a further aspect, the invention relates to a method of treating a cancer patient, said method comprising
a. assessing if the cancer patient is a responder to treatment with chemotherapy by the method of the invention and
b. (i) treating the cancer patient with chemotherapy if the patient has a reduced risk for not being a responder to treatment with chemotherapy or (ii) treating the cancer patient with a treatment regimen which comprises a treatment which is different from chemotherapy if the patient has an increased risk for not being a responder to treatment with chemotherapy, wherein said treatment regimen preferably does not comprise chemotherapy.

In one embodiment, the treatment regimen comprises a treatment with an antibody acting through recruiting the patient's immune system to destroy tumor cells.

In a further aspect, the invention relates to a method of assessing the clinical outcome for a cancer patient, said method comprising determining the level of cells of the macrophage lineage in a tumor sample obtained from the patient. In one embodiment, the cancer patient has a HER2-positive tumor.

In one embodiment, the presence of cells of the macrophage lineage or a level of cells of the macrophage lineage which is higher than a defined threshold indicates an increased risk of poor clinical outcome. In one embodiment, the absence of cells of the macrophage lineage or a level of cells of the macrophage lineage which is lower than a defined threshold indicates a reduced risk of poor clinical outcome.

In one embodiment, the level of cells of the macrophage lineage is determined by determining the expression level of one or more macrophage specific markers, wherein the one or more macrophage specific markers preferably are selected from the group consisting of CD68, CD14, CD163, CD80, MAC2 (galectin-3), EMR1-F4/80, CD11b and CD205.

In a further aspect, the invention relates to a method of assessing the clinical outcome for a cancer patient, said method comprising determining the expression level of CD68 in a tumor sample obtained from the patient. In one embodiment, the cancer patient has a HER2-positive tumor.

In one embodiment, expression of CD68 or an expression level of CD68 which is higher than a defined expression threshold indicates an increased risk of poor clinical outcome. In one embodiment, no expression of CD68 or an expression level of CD68 which is lower than a defined expression threshold indicates a reduced risk of poor clinical outcome.

In one embodiment of the above aspects of the invention, assessing the clinical outcome for a cancer patient comprises predicting the likelihood of survival, recurrence-free survival, and distant recurrence-free survival.

In one embodiment of the above aspects of the invention, poor clinical outcome comprises a relative reduction in one or more of survival, recurrence-free survival, and distant recurrence-free survival.

In one embodiment of the above aspects of the invention, the tumor is a solid tumor. In one embodiment of the above aspects of the invention, the tumor is a breast tumor. In one embodiment of the above aspects of the invention, the cancer is breast cancer.

In one embodiment of the above aspects of the invention, determining the expression level comprises determining the expression level of RNA transcript and/or the expression level of protein. In one embodiment of the above aspects of the invention, the expression level is normalized against the (mean) expression level of one or more housekeeping genes in said tumor sample.

In a further aspect, the present invention relates to a kit comprising means for determining the level of one or more macrophage markers such as CD68 in a sample isolated from a patient. In one embodiment, said kit is useful for conducting the methods of all aspects of the present invention. In one embodiment, said kit further comprises a data carrier. In one preferred embodiment, said data carrier is an electronical or a non-electronical data carrier. In one embodiment, said data carrier comprises instructions on how to carry out the methods of all aspects of the invention.

Other objects, advantages and features of the present invention will become apparent from the following detailed description when considered in conjunction with the accompanying figures.

### BRIEF DESCRIPTION OF THE FIGURES

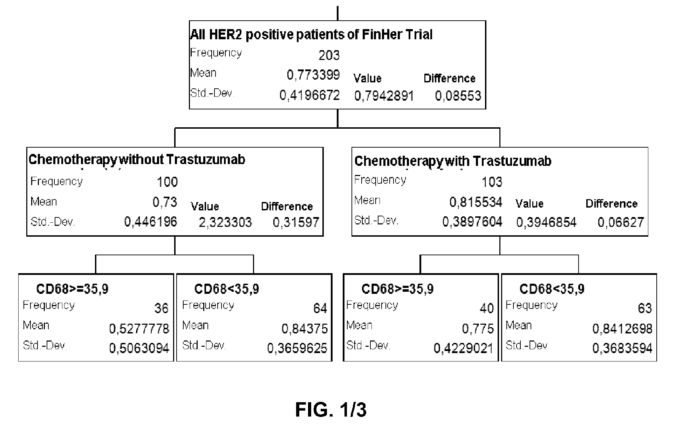

**Figure 1****: Distant Metastasis Free Survival of patients according to CD68 mRNA status**
   A partitioning test is shown to evaluate the prognostic and predictive value of CD68 mRNA Available data from 867 tumors were first stratified by HER2 status to identify HER2 positive tumors ("HER2 Status>=1"). Within this subgroup CD68 mRNA distinguishes a Distant Metastasis Free Survival ("DMFS") of 69% if CD68 mRNA expression is above the expression of 35,9 (n=123 patients) versus 90 percent if CD68 mRNA expression is below the median expression of 35,9 (n=80patients). This shows that high expression of CD68 mRNA is prognostic for chemotherapy treated HER2 positive tumors with high expression of CD68 being associated with inferior survival. Further Stratification by trastuzumab treatment status identifies similar good survival of tumors having low CD68 mRNA expression (below 35,9) with - 84% Distant Metastasis Free Survival ("DMFS") irrespective of trastuzumab treatment,, whereas tumors exhibiting high expression of CD68 (above or equal to 35,9) have significantly different DMFS . HER2 positive tumors treated with trastiuzumab in addition to adjuvant chemotherapy having better DMFS of 78%, while tumors receiving no trastuzumab have worde outcome (53% DMFS).

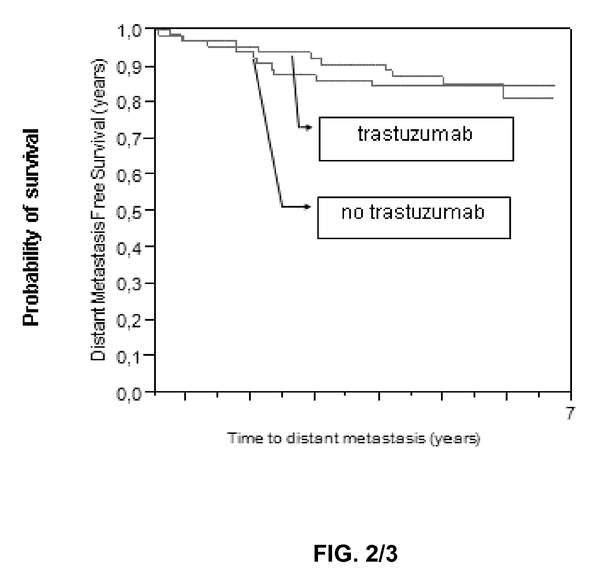

**Figure 2****: Distant Metastasis Free Survival of HER2 positive CD68 negatove tumor patients according to trastuzumab treatment**
   Distant Metastasis Free Survival ("DMFS") by Kaplan Meier analysis of HER2 positive tumors exhibiting high CD68 levels depending on the treatment with or without trastuzumab. Addition of trastuzumab does not result in improved survival at higher than the median CD68 mRNA expression levels. The 3 year DMFS is at about 90%.

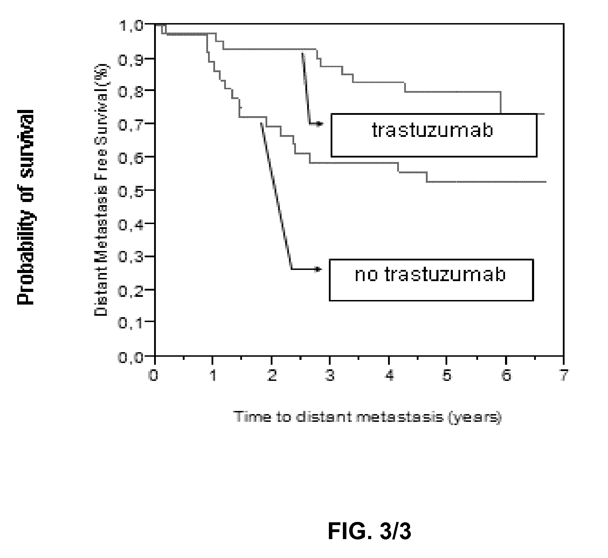

**Figure 3****: Distant Metastasis Free Survival of HER2 positive CD68 negative tumor patients according to trastuzumab treatment**
   Distant Metastasis Free Survival ("DMFS") by Kaplan Meier analysis of HER2 positive tumors exhibiting high CD68 levels depending on the treatment with or without trastuzumab. Addition of trastuzumab results in significantly improved survival at CD68 mRNA levels higher than 35,9 Without trastuzumab treatment the median DMFS survival after 3 years is only at about 70%, while the addition of the anti-HER2 antibody in HER2 positive tumors with lower levels of CD68 positive cells significantly increases the 3-year DMFS to 95%.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

The practice of the present invention will employ, unless otherwise indicated, conventional methods of chemistry, biochemistry, cell biology, immunology, and recombinant DNA techniques which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention. Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention.

The present inventors provide a test to measure the eligibility of patients for certain cancer treatments, in particular antibody therapy or chemotherapy, and to draw conclusions on the prognosis of a cancer patient. The results obtained using these tests enable the physician to decide on a suitable treatment for a cancer patient, and, in particular, to decide whether antibody therapy, chemotherapy, or both should be administered to a particular cancer patient.

The macrophage lineage comprises a system of cells of large diversity with respect to their morphological, functional, and metabolic properties. This distinguishes these cells from other myeloid cells, which are relatively homogeneous, such as neutrophils. The large diversity of the macrophage lineage is formed under the influence of growth factors.

The term "macrophage" refers to a cell of the macrophage lineage produced by the differentiation of monocytes. Both macrophages and monocytes are phagocytes, acting in both non-specific defense (innate immunity) as well as to help initiate specific defense mechanisms (adaptive immunity) of vertebrates. Human macrophages are about 21 µm in diameter. Macrophages are specialized phagocytic cells that attack foreign substances, infectious microbes and cancer cells through destruction and ingestion. They also stimulate lymphocytes and other immune cells to respond to pathogens. Macrophages can be classified into two main groups designated M1 and M2. M1 macrophages, or classically activated macrophages, are immune effector cells that are aggressive against microbes and can engulf and digest affected cells much more readily, and they also produce many lymphokines. M1 macrophages are activated by LPS and IFN-gamma, and secrete high levels of IL-12 and low levels of IL-10. M2 describes other types of macrophages, including those that function in wound healing and tissue repair, and those that turn off immune system activation by producing anti-inflammatory cytokines like IL-10. M2, or alternatively activated macrophages, are activated by IL-4 and produce high levels of IL-10 and low levels of IL-12. Tumor-associated macrophages are thought to be M2 macrophages. In one embodiment of the invention, macrophages are M2 macrophages. Macrophages can be identified and their level determined by detecting or determining the level of proteins specifically expressed in macrophages including but not limited to CD68, CD14, CD163, CD80, MAC2 (galectin-3), EMR1-F4/80, CD11b and CD205, or RNA encoding these proteins. CD68 (Cluster of Differentiation 68) which according to the invention is a particularly preferred marker is a 110-kD transmembrane glycoprotein that is highly expressed by human tissue macrophages. CD68 is a member of a family of hematopoietic mucin-like molecules that includes leukosialin/CD43 and stem cell antigen CD34. The CD68 gene is mapped to 17p13.1. Immunohistochemistry can be used to identify the presence of CD68, which is found in the cytoplasmic granules of a range of different blood cells. It is particularly useful as a marker for the various cells of the macrophage lineage, including monocytes, histiocytes, giant cells, Kupffer cells, and osteoclasts.

The term "CD68" preferably relates to human CD68, and, in particular, to a protein comprising, preferably consisting of the amino acid sequence of SEQ ID NO: 1 of the sequence listing or a variant of said amino acid sequence. According to the invention, the term "CD68" is also used to include nucleic acid, in particular RNA, encoding CD68 protein, in particular, a protein comprising, preferably consisting of the amino acid sequence of SEQ ID NO: 1 of the sequence listing or a variant of said amino acid sequence. According to the invention, the term "CD68" includes a nucleic acid sequence comprising, preferably consisting of the nucleic acid sequence of SEQ ID NO: 2.

The term "marker" refers to a biological molecule, e.g., a nucleic acid, in particular RNA, or protein, whose presence or amount can be detected and correlated with a known condition, such as a disease state, or the development of a disease. In particular, the term "marker" as used herein, refers to a differentially expressed biological molecule which may be utilized to make a prediction, prognosis or diagnosis.

"Prognosis" as used herein refers to a prediction of outcome and, in particular, the probability of progression-free survival (PFS) or distant metastasis free survival (DFS). Survival is usually calculated as an average number of months (or years) that 50% of patients survive, or the percentage of patients that are alive after 1, 5, 15, and 20 years. Prognosis is important for treatment decisions because patients with a good prognosis are usually offered less invasive treatments, while patients with poor prognosis are usually offered more aggressive treatment, such as more extensive chemotherapy drugs.

"Prediction" as used herein refers to providing information about the possible response of a disease to a distinct therapeutic treatment.

"Differential expression" as used herein, refers to both quantitative as well as qualitative differences in expression patterns observed in at least two different individuals or samples taken from individuals.

The methods of the present invention can be carried out with sample material. Said sample material, may be isolated from a patient, e.g. from the human body. Subsequently, the sample material can be fractionated and/or purified. It is, for example, possible to store the sample material to be tested in a freezer and to carry out the methods of the present invention at an appropriate point in time after thawing the respective sample material. The term "sample", as used herein, refers to any biological sample which may be isolated from a patient and used for analysis purposes, in particular for the determination of the level of cells of the macrophage lineage and/or for determining the expression level of CD68. Said sample may be a body fluid sample, a tissue sample, or a cell sample. For example, samples encompassed by the present invention are tissue (e.g. section or explant) samples, single cell samples, cell colony samples, cell culture samples, blood (e.g. whole blood or blood fraction such as blood cell fraction, serum or plasma) samples, urine samples, or samples from other peripheral sources. In one particularly preferred embodiment, the sample is a tissue sample (e.g., a biopsy from a subject with or suspected of having cancerous tissue). More preferably, the sample is a biopsy of a tumor, most preferably the sample is fixed tumor tissue such as a formalin fixed paraffin embedded (FFPE) tumor sample. The sample may be obtained from a patient prior to initiation of a therapeutic treatment, during the therapeutic treatment, and/or after the therapeutic treatment, e.g. prior to, during or following the administration of cancer therapy.

The term "patient", as used herein, refers to any organism such as vertebrate, particularly any mammal, including both a human and another mammal, e.g. an animal such as a rodent, a rabbit, or a monkey. The rodent may be a mouse, rat, hamster, guinea pig, or chinchilla. Preferably, the patient is a human.

Various methods known in the art can be used to determine the level of cells of the macrophage lineage. Preferably, the level of cells of the macrophage lineage is determined by determining the level of one or more markers of cells of the macrophage lineage (macrophage markers or macrophage specific markers) such as CD68, CD14, CD163, CD80, MAC2 (galectin-3), EMR1-F4/80, CD11b and CD205, preferably CD68, or RNA coding therefor. In one embodiment, the level of one or more markers of cells of the macrophage lineage is the expression level. The expression level of one or more markers of cells of the macrophage lineage and, in particular, the expression level of CD68 can be determined by any suitable method. In one embodiment, macrophages may be bound with one or more labelled antibodies against one or more markers prior to determination of the level.

The term "expression level" (or simply "level"), as used herein, refers to the expression of a particular gene so as to produce transcript and/or protein. The expression level may be determined on the mRNA level (transcriptional level) or protein level (translational level), for example, by measuring the transcribed mRNA (e.g. via northern blot), by measuring the produced protein (e.g. via Western Blot), by directly or indirectly staining the protein (e.g. via immunohistochemistry) or by directly staining the mRNA (e.g. via *in situ* hybridization).

Said expression level may be indicated as concentration of mRNA or protein, amount of mRNA or protein or extinction units such as relative fluorescence units.

In one preferred embodiment, the expression level is determined with an immunoassay, gel electrophoresis, spectrometry, chromatography, *in situ* hybridization, or a combination thereof. In preferred embodiments,
(i) the immunoassay is an enzyme immunoassay, preferably an enzyme-linked immunosorbent assay (ELISA), a Western Blot (immunoblot), a radio immunoassay (RIA), or a luminescence immunoassay (LIA),
(ii) the gel electrophoresis is 1D or 2D gel electrophoresis,
(iii) the spectrometry is mass spectrometry (MS), preferably tandem mass spectrometry (MS/MS),
(iv) the chromatography is liquid chromatography (LC) or affinity chromatography,
(v) the *in situ* hybridization is a silver *in situ* hybridization (SISH), chromogenic *in situ* hybridization (CISH), or fluorescence *in situ* hybridization (FISH),
(vi) the chromatography is combined with spectrometry, preferably mass spectrometry (MS), and is more preferably liquid chromatography-mass spectrometry (LC-MS) and most preferably liquid chromatography-tandem mass spectrometry (LC-MS/MS), or
(vii) the gel electrophoresis is combined with an immunoassay and is more preferably a 2D immunoblot.

In one embodiment, the mass spectrometry is an electrospray ionization mass spectrometry (ESI-MS), a matrix-assisted laser desorption/ionization mass spectrometry (MALDI-MS), or an electron capture dissociation mass spectrometry (ECD-MS).

The term "1D (One-dimensional) gel electrophoresis", as used herein, includes protein separation techniques such as Sodium dodecyl sulfate polyacrylamide gel electrophoresis (SDS-PAGE), native gel electrophoresis and isoelectric focusing. The SDS-PAGE, for example, is a technique for separating proteins based on their ability to move within an electrical current, which is a function of the length of their polypeptide chains or of their molecular weight. The addition of the SDS detergent to these samples gives the proteins the same electrical charge. SDS-PAGE allows for separation of proteins from a wide range of samples including cells, tissues and whole blood. Native Gel Electrophoresis, for example, is a technique used mainly in protein electrophoresis where the proteins are not denatured and therefore separated based on their charge-to-mass ratio. The main types of native gels used in protein electrophoresis are polyacrylamide gels and agarose gels. It should be noted that unlike SDS-PAGE type electrophoreses, native gel electrophoresis does not use a charged denaturing agent. The proteins being separated, therefore, differ in molecular mass and intrinsic charge and experience different electrophoretic forces dependent on the ratio of the two. Since the proteins remain in the native state they may be visualized not only by general protein staining reagents but also by specific enzyme-linked staining.

The term "2D (Two-dimensional) gel electrophoresis", as used herein, refers to a form of gel electrophoresis commonly used to analyze proteins in two dimensions. 2D gel electrophoresis, for example, begins with 1D electrophoresis but then separates the molecules by a second property in a direction 90 degrees from the first. In 1D electrophoresis, proteins are separated in one dimension, so that all the proteins/molecules will lie along a lane but that the molecules are spread out across a 2D gel. The two dimensions that proteins are separated into using this technique can be isoelectric point, protein complex mass in the native state, and protein mass. Preferably, the first dimension is isoelectric focusing (IEF) and the second dimension is SDS-PAGE.

The term "isoelectric focusing (IEF) (also known as electrofocusing)", as used herein, relates to a technique in order to separate the proteins by their isoelectric point. Thereby, a gradient of pH is applied to a gel and an electric potential is applied across the gel, making one end more positive than the other. At all pH-stages other than the isoelectric point, proteins will be charged. If the proteins are positively charged, they will be pulled towards the more negative end of the gel and if the proteins are negatively charged they will be pulled to the more positive end of the gel. The proteins applied in the IEF will move along the gel and will accumulate at their isoelectric point; that is, the point at which the overall charge on the protein is 0 (a neutral charge). IEF is preferably carried out using immobilized pH gradient (iPG) gels, or iPG strips, more preferably dry and rehydratable iPG strips. Microfluidic chip based isoelectric focusing may also be used (Sommer and Hatch, Electrophoresis. 2009 Mar;30(5):742-5.).

The term "Western blotting", as used herein, relates to a technique which allows the detection of specific proteins (native or denatured) from extracts made from cells or tissues or body liquid samples, before or after any purification steps. Proteins are generally separated by size using gel electrophoresis before being transferred to a synthetic membrane (typically nitrocellulose or PVDF) via dry, semi-dry, or wet blotting methods. The membrane can then be probed using antibodies using methods similar to immunohistochemistry, but without a need for fixation. Detection is typically performed using reporter enzyme linked antibodies, e.g. peroxidase linked antibodies to catalyze a chemiluminescent reaction or alkaline phosphatase linked antibodies to catalyze a colorimetric reaction. Western blotting is a routine molecular biology method that can be used to semi-quantitatively or quantitatively compare protein levels between extracts. The size separation prior to blotting allows the protein molecular weight to be gauged as compared with known molecular weight markers. Western blotting is an analytical technique used to detect specific proteins in a given sample of tissue homogenate or extract. It uses gel electrophoresis to separate proteins by the length of the polypeptide (denaturing conditions) or by the 3-D structure of the protein (native/ non-denaturing conditions). The colorimetric detection method may depend on incubation of the Western blot with a substrate that reacts with the reporter enzyme (such as peroxidase) that is bound to the secondary antibody. This converts the soluble dye into an insoluble form of a different color that precipitates next to the enzyme and thereby stains the membrane. Development of the blot may be then stopped by washing away the soluble dye. Protein levels may be evaluated through densitometry (how intense the stain is) or spectrophotometry. Further, chemiluminescent detection methods may depend on incubation of the Western blot with a substrate that will luminesce when exposed to the reporter on the secondary antibody. The light is then detected by photographic film, and more recently by CCD cameras which capture a digital image of the Western blot. The image may be analysed by densitometry, which evaluates the relative amount of protein staining and quantifies the results in terms of optical density. Newer software allows further data analysis such as molecular weight analysis if appropriate standards are used.

The term "enzyme-linked immunosorbent assay" or "ELISA", as used herein, relates to a method for quantitatively or semi-quantitatively determining protein concentrations from a sample, e.g. blood plasma, serum or cell/tissue extracts, in a multi-well plate format (usually 96-wells per plate). Broadly, proteins in solution are adsorbed to ELISA plates. Antibodies specific for the protein of interest are used to probe the plate. Background is minimized by optimizing blocking and washing methods (as for IHC), and specificity is ensured via the presence of positive and negative controls. Detection methods are usually colorimetric or chemiluminescence based.

The term "mass spectrometry (MS)", as used herein, refers to the use of an ionization source to generate gas phase ions from a sample on a surface and detecting the gas phase ions with a mass spectrometer. The term "laser desorption mass spectrometry" refers to the use of a laser as an ionization source to generate gas phase ions from a sample on a surface and detecting the gas phase ions with a mass spectrometer. The mass spectrometry may be a matrix-assisted laser desorption/ionization mass spectrometry or MALDI. In MALDI, the analyte is typically mixed with a matrix material that, upon drying, co-crystallizes with the analyte. The matrix material absorbs energy from the energy source which otherwise would fragment the labile biomolecules or analytes. The mass spectrometry may also be a surface-enhanced laser desorption/ionization mass spectrometry or SELDI. In SELDI, the surface on which the analyte is applied plays an active role in the analyte capture and/or desorption.

The term "tandem mass spectrometry (MS/MS)", as used herein, refers to multiple rounds of mass spectrometry, usually separated by some form of molecule fragmentation. For example, one mass analyzer can isolate one peptide from many entering a mass spectrometer. A second mass analyzer then stabilizes the peptide ions while they collide with a gas, causing them to fragment by collision-induced dissociation (CID). A third mass analyzer then sorts the fragments produced from the peptides. Tandem MS can also be done in a single mass analyzer over time, as in a quadrupole ion trap. There are various methods for fragmenting molecules for tandem MS, including collision-induced dissociation (CID), electron capture dissociation (ECD), electron transfer dissociation (ETD), infrared multiphoton dissociation (IRMPD), blackbody infrared radiative dissociation (BIRD), electron-detachment dissociation (EDD) and surface-induced dissociation (SID).

In one particularly preferred embodiment, the expression level is determined using a method involving reverse transcription quantititative polymerase chain reaction (qPCR or RT-qPCR). In one embodiment, the method comprises the use of target-specific primers such as CD68-specific primers having a length of 15 to 30 nucleotides.

In one aspect, the present invention relates to a method of or involves steps of determining the expression level of CD68 using CD68-specific primers comprising at least 10, preferably at least 15 contiguous nucleotides of the sequences of SEQ ID NOs: 5 and 6, respectively. In one embodiment, such CD68-specific primers may comprise the sequences of SEQ ID NOs: 5 and 6, respectively. Alternatively or additionally, in one embodiment, the method or steps comprise the use of a CD68-specific probe e.g. having a length of 20 to 35 nucleotides. Said probe may comprise at least 15, preferably at least 20 contiguous nucleotides of the sequence of SEQ ID NO: 7.

In one embodiment, the expression level is normalized against the (mean) expression level of one or more reference genes.

In one embodiment, the one or more reference genes are selected from the group comprising CALM2, B2M, RPL37A, GUSB, HPRT1 and GAPDH.

In one embodiment of the present invention, it is determined whether the level of cells of the macrophage lineage is above and/or below a threshold value. In one particular embodiment of the present invention, it is determined whether the expression level of CD68 is above and/or below a threshold value. Said threshold value may be indicative for a cancer patient being a responder or non-responder to antibody treatment and/or treatment with chemotherapy and/or for the clinical outcome of the cancer patient.

A level of cells of the macrophage lineage and/or an expression level of CD68 which is above a threshold value preferably is indicative for a cancer patient being a responder to antibody treatment. A level of cells of the macrophage lineage and/or an expression level of CD68 which is below a threshold value preferably is indicative for a cancer patient being a non-responder to antibody treatment.

A level of cells of the macrophage lineage and/or an expression level of CD68 which is above a threshold value preferably is indicative for a cancer patient being a responder to chemotherapy treatment. A level of cells of the macrophage lineage and/or an expression level of CD68 which is below a threshold value preferably is indicative for a cancer patient being a non-responder to chemotherapy treatment.

A level of cells of the macrophage lineage and/or an expression level of CD68 which is above a threshold value preferably is indicative for a poor clinical outcome of the cancer patient. A level of cells of the macrophage lineage and/or an expression level of CD68 which is below a threshold value preferably is indicative for a favourable clinical outcome of the cancer patient.

In the context of the present invention, the term "threshold" or "threshold value" may belong to the mean cut-off value calculated from a number of samples, said number of samples being obtained from a number of subjects, in particular, subjects having cancer. To obtain the "threshold" said number of subjects may include responders and/or non-responders to antibody treatment and/or responders and/or non-responders to chemotherapy treatment and/or subjects afflicted with cancer having or having had poor and/or favourable clinical outcome. Said threshold may represent an amount or a concentration of cells of the macrophage lineage and/or of one or more markers of cells of the macrophage lineage. In particular, said threshold may represent an amount or a concentration of CD68.

The term "subject", as used herein, relates to any organism such as vertebrate, particularly any mammal, including both a human and another mammal, e.g. an animal such as a rodent, a rabbit, or a monkey. The rodent may be a mouse, rat, hamster, guinea pig, or chinchilla. Preferably, the subject is a human. In one embodiment, a subject is a subject with or suspected of having a disease, in particular cancer, also designated "patient" herein.

In embodiments of the present invention, where the level of cells of the macrophage lineage and/or the expression level of CD68 is above a threshold value indicative for
(i) responsiveness to antibody treatment, the threshold value is preferably the upper value of the 95% confidence interval and belonging to the mean value calculated from subjects being non-responders to antibody treatment,
(ii) responsiveness to chemotherapy treatment, the threshold value is preferably the upper value of the 95% confidence interval and belonging to the mean value calculated from subjects being responders to chemotherapy treatment,
(iii) poor clinical outcome, the threshold value is preferably the upper value of the 95% confidence interval and belonging to the mean value calculated from subjects having and/or having had poor clinical outcome;
and/or
where the level of cells of the macrophage lineage and/or the expression level of CD68 is below a threshold value indicative for
(i) non-responsiveness to antibody treatment, the threshold value is preferably the lower value of the 95% confidence interval and belonging to the mean value calculated from subjects being responders to antibody treatment,
(ii) non-responsiveness to chemotherapy treatment, the threshold value is preferably the lower value of the 95% confidence interval and belonging to the mean value calculated from subjects being non-responders to chemotherapy treatment,
(iii) favourable clinical outcome, the threshold value is preferably the lower value of the 95% confidence interval and belonging to the mean value calculated from subjects having and/or having had favourable clinical outcome.

The 95% confidence interval and the mean value may be determined by techniques known in the art.

For the determination of the mean value, at least two subjects, preferably at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects, are tested.

In one embodiment of the present invention, the level of cells of the macrophage lineage and/or the expression level of CD68 is compared to a reference level of cells of the macrophage lineage and/or a reference expression level of CD68.

Said reference level may be a level which may be determined by measuring one or more reference samples, such as at least 1, at least 2, at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 reference samples, from one or more subjects, such as at least 1, at least 2, at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects. Preferably, one reference sample per subject is measured. It is further preferred that at least two subjects are tested. Said subjects may be subjects being non-responders to antibody treatment, subjects being responders to chemotherapy treatment, subjects having and/or having had poor clinical outcome, subjects being responders to antibody treatment, subjects being non-responders to chemotherapy treatment and/or subjects having and/or having had favourable clinical outcome.

Said reference level may be an average level (or mean level).

The average level may be determined by measuring one reference sample from each one of the one or more subjects, preferably at least 5, at least 10, at least 20, at least 30, at least 40, at least 50, at least 60, at least 70, at least 80, at least 90, at least 100, at least 200, at least 300, at least 400, at least 500, at least 600, at least 700, at least 800, at least 900, at least 1000, at least 1500, or at least 2000 subjects, and calculating the middle value of the reference levels determined therein.

In the context of the present invention, the term "reference sample" refers to any biological sample which is used for comparison purposes, in particular for comparison with the "sample" to be tested. Statements made with regard to the preferred embodiments of the "sample" fully analogously apply to the preferred embodiments of the "reference sample". It is preferred that the source and the amount of the "sample" as well as of the "reference sample" are the same.

The term "a level which is higher than", as used herein, means that the level is preferably by at least 5%, by at least 10%, by at least 20%, by at least 30%, by at least 40%, by at least 50%, by at least 60%, by at least 70%, by at least 80%, by at least 90%, by at least 100%, by at least 110%, by at least 120%, by at least 130%, by at least 140%, by at least 150%, by at least 200%, by at least 250%, by at least 300%, by at least 400%, or by at least 500% higher compared to the reference level.

The term "a level which is lower than", as used herein, means that the level is preferably by at least 5%, by at least 10%, by at least 20%, by at least 30%, by at least 40%, by at least 50%, by at least 60%, by at least 70%, by at least 80%, by at least 90%, by at least 100%, by at least 110%, by at least 120%, by at least 130%, by at least 140%, by at least 150%, by at least 200%, by at least 250%, by at least 300%, by at least 400%, or by at least 500% lower compared to the reference level.

In one embodiment, a method of the invention is performed on a patient who is already diagnosed as having cancer.

The present invention further relates to a kit comprising means such as reagents for determining the level of cells of the macrophage lineage in a sample isolated from a patient. In particular, the present invention relates to a kit comprising means such as reagents for determining the expression level of one or more markers of cells of the macrophage lineage in a sample isolated from a patient. In one embodiment, the present invention relates to a kit comprising means such as reagents for determining the expression level of CD68 in a sample isolated from a patient. Said kit is useful for conducting the methods of the present invention.

In the context of the present invention, the term "kit of parts (in short: kit)" is understood to be any combination of at least some of the components identified herein, which are combined, coexisting spatially, to a functional unit, and which can contain further components.

In one embodiment, the kit comprises one or more means that specifically bind to (a) marker protein(s) of cells of the macrophage lineage or marker mRNA of cells of the macrophage lineage.

Said means that specifically bind to (a) marker protein(s) of cells of the macrophage lineage may be antibodies or fragments thereof, which are capable of specially binding to an epitope or a suitable structural element of a protein to be detected such as markers of cells of the macrophage lineage, e.g. CD68. Said antibody fragments may be Fab or F(ab)2 fragments.

In other embodiments, said means may be nucleic acid. For nucleic acid detection, the kits generally comprise (but are not limited to) probes specific for marker mRNA of cells of the macrophage lineage. For Quantitative PCR, the kits generally comprise pre-selected primers specific for marker nucleic acid sequences of cells of the macrophage lineage The Quantitative PCR kits may also comprise enzymes suitable for amplifying nucleic acids (e.g., polymerases such as Taq), and deoxynucleotides and buffers needed for the reaction mixture for amplification. The Quantitative PCR kits may also comprise probes specific for marker nucleic acid sequences of cells of the macrophage lineage. In some embodiments, the Quantitative PCR kits also comprise components suitable for reverse-transcribing RNA including enzymes (e.g. reverse transcriptases) and primers for reverse transcription along with deoxynucleotides and buffers needed for the reverse transcription reaction.

In certain embodiments, said means are detectably labelled.

In certain embodiments the kit comprises a package insert allowing the analysis of results obtained with said kit.

The antibody or antibody fragment may be bound to a solid support, e.g. a plastic surface, to allow binding and detection of a protein. For example, a microtiter plate can be used as a plastic surface. The detection of the binding can be effected by using a secondary antibody labeled with a detectable group. The detectable group can be an enzyme like horseradish peroxidase (HRP) or alkaline phosphatase detectable by adding a suitable substrate to produce a colour or a fluorescence signal.

Said kit may further comprise (i) a container, and/or (ii) a data carrier. Said container may be filled with one or more of the above mentioned means or reagents. Said data carrier may be a non-electronical data carrier, e.g. a graphical data carrier such as an information leaflet, an information sheet, a bar code or an access code, or an electronical data carrier such as a floppy disk, a compact disk (CD), a digital versatile disk (DVD), a microchip or another semiconductor-based electronical data carrier. The access code may allow the access to a database, e.g. an internet database, a centralized, or a decentralized database. Said data carrier may comprise instructions for the use of the kit in the methods of the invention.

Additionally or alternatively, said kit may comprise materials desirable from a commercial and user standpoint including buffer(s), reagent(s) and/or diluent(s) for determining the level of protein or mRNA.

The above mentioned data carrier may comprise a threshold value or reference level of a protein or mRNA. In case that the data carrier comprises an access code which allows the access to a database, said threshold value or reference level is deposited in this database.

In addition, the data carrier may comprise information or instructions on how to carry out the methods of the present invention.

Based on the results obtained (i.e. on the basis of the level of cells of the macrophage lineage, in particular on the basis of the expression level of one or more markers of cells of the macrophage lineage, specifically on the basis of the expression level of CD68), the medical practitioner may choose a cancer therapy, in particular antibody therapy or chemotherapy, or both, to which the patient is predicted as being responsive. Preferably, a cancer therapy to which the patient is predicted as being non-responsive is not administered to the patient.

Based on the result that the level of cells of the macrophage lineage, in particular the expression level of one or more markers of cells of the macrophage lineage, specifically the expression level of CD68 is high, in particular higher than a defined threshold, the medical practitioner may choose to administer cancer therapy which is different from antibody therapy, in particular antibody therapy acting through recruiting the patient's immune system to destroy tumor cells. In particular, the medical practitioner may choose to administer chemotherapy.

Based on the result that the level of cells of the macrophage lineage, in particular the expression level of one or more markers of cells of the macrophage lineage, specifically the expression level of CD68 is low, in particular lower than a defined threshold, the medical practitioner may choose to administer antibody therapy, in particular antibody therapy acting through recruiting the patient's immune system to destroy tumor cells, optionally in combination with chemotherapy.

The term "(therapeutic) treatment", in particular in connection with the treatment of cancer as used herein, relates to any treatment which improves the health status and/or prolongs (increases) the lifespan of a patient. Said treatment may eliminate cancer, reduce the size or the number of tumors in a patient, arrest or slow the development of cancer in a patient, inhibit or slow the development of new cancer in a patient, decrease the frequency or severity of symptoms in a patient, and/or decrease recurrences in a patient who currently has or who previously has had cancer.

Adjuvant therapy is a treatment that is given in addition to the primary, main or initial treatment. The surgeries and complex treatment regimens used in cancer therapy have led the term to be used mainly to describe adjuvant cancer treatments. An example of adjuvant therapy is the additional treatment usually given after surgery where all detectable disease has been removed, but where there remains a statistical risk of relapse due to occult disease.

Terms such as "responsive", or "responder" refer, in a therapeutic setting, to the fact that a patient has a therapeutic benefit from a given mode of treatment and, in particular, to the observation of an alleviation, prevention or elimination of a disease including shortening the duration of a disease, arresting or slowing progression or worsening of a disease, inhibiting or slowing the development of a new disease and/or recurrences, preventing or delaying the onset of a disease or the symptoms thereof, decreasing the frequency or severity of symptoms in a patient who currently has or who previously has had a disease and/or prolonging the lifespan of the patient. In particular, they refer to the observation of a reduction in tumor mass or of an increase in tumor free time, recurrence free time or overall survival time.

Terms such as "non-responsive" or "non-responder" refer, in a therapeutic setting, to the fact that a patient has no therapeutic benefit from a given mode of treatment and, in particular, to no observation of an alleviation, prevention or elimination of a disease, i.e. the patient is resistant to treatment.

Complete response is defined as the absence of any residual disease such as cancer, and is usually assessed by pathological analysis of acquired tissue samples. In this context, the term "pathological complete response" (pCR) is frequently used. In particular, pCR is defined as the absence of any residual invasive tumour cells in the original tumor bed. However, the definition of pCR may vary between different grading systems. Pathological complete response has shown to be a prognostic factor for overall better survival, but also for disease-free survival and recurrence free survival.

Recurrence-free survival is defined as the time from randomization to the first of either recurrence or relapse, second cancer, or death.

The term "clinical outcome" is defined as the clinical result of a disease, e.g. reduction or amelioration of symptoms, in particular following a treatment.

The term "recurrence" with respect to cancer includes occurrence of tumor cells at the same site and organ of the origin disease, distant metastasis that can appear even many years after the initial diagnosis and therapy of cancer, or to local events such as infiltration of tumor cells into regional lymph nodes.

By "treat" is meant to administer a treatment such as a compound or composition or a combination of compounds or compositions to a subject in order to alleviate, prevent or eliminate a disease.

In the context of the present invention, terms such as "protect" or "prevent" relate to the prevention of the occurrence and/or the propagation of a disease in a subject and, in particular, to minimizing the chance that a subject will develop a disease or to delaying the development of a disease. For example, a subject at risk for cancer would be a candidate for therapy to prevent cancer.

By "being at risk" is meant a subject that is identified as having a higher than normal chance of developing a disease, in particular cancer, compared to the general population. In addition, a subject who has had, or who currently has, a disease, in particular cancer, is a subject who has an increased risk for developing a disease, as such a subject may continue to develop a disease. Subjects who currently have, or who have had, a cancer also have an increased risk for cancer metastases.

As used herein, the term "combination" in the context of the administration of a therapy refers to the use of more than one therapy or therapeutic agent. The use of the term "in combination" does not restrict the order in which the therapies or therapeutic agents are administered to a subject. A therapy or therapeutic agent can be administered prior to, concomitantly with, or subsequent to the administration of a second therapy or therapeutic agent to a subject. Preferably, the therapies or therapeutic agents are administered to a subject in a sequence, amount and/or within a time interval such that the therapies or therapeutic agents can act together. In a particular embodiment, the therapies or therapeutic agents are administered to a subject in a sequence, amount and/or within a time interval such that they provide an increased benefit than if they were administered otherwise, in particular, independently from each other. Preferably, the increased benefit is a synergistic effect.

According to the invention, the term "disease" refers to any pathological state, including cancer, in particular those forms of cancer described herein. Any reference herein to cancer or particular forms of cancer also includes cancer metastasis thereof. In a preferred embodiment, a disease to be treated according to the present application involves cells expressing a tumor antigen such as HER2/neu.

"Disease involving cells expressing a tumor antigen" means according to the invention that a tumor antigen is expressed in cells of a diseased tissue or organ. In one embodiment, expression of a tumor antigen in cells of a diseased tissue or organ is increased compared to the state in a healthy tissue or organ. An increase refers to an increase by at least 10%, in particular at least 20%, at least 50%, at least 100%, at least 200%, at least 500%, at least 1000%, at least 10000% or even more. In one embodiment, expression is only found in a diseased tissue, while expression in a corresponding healthy tissue is repressed. According to the invention, diseases involving cells expressing a tumor antigen include cancer diseases. Furthermore, according to the invention, cancer diseases preferably are those wherein the cancer cells express a tumor antigen.

As used herein, a "cancer disease" or "cancer" includes a disease characterized by aberrantly regulated cellular growth, proliferation, differentiation, adhesion, and/or migration. By "cancer cell" is meant an abnormal cell that grows by a rapid, uncontrolled cellular proliferation and continues to grow after the stimuli that initiated the new growth cease. Preferably, a "cancer disease" is characterized by cells expressing a tumor antigen.

The term "cancer" according to the invention comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the metastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases.

In one particularly preferred embodiment, the term "cancer" according to the invention relates to "breast cancer" and/or "breast cancer metastasis".

The term "breast cancer" relates to a type of cancer originating from breast tissue, most commonly from the inner lining of milk ducts or the lobules that supply the ducts with milk. Cancers originating from ducts are known as ductal carcinomas, while those originating from lobules are known as lobular carcinomas. Occasionally, breast cancer presents as metastatic disease. Common sites of metastasis include bone, liver, lung and brain. Breast cancer occurs in humans and other mammals. While the overwhelming majority of human cases occur in women, male breast cancer can also occur.

Treatment of breast cancer may include surgery, medications (hormonal therapy and chemotherapy), radiation and/or immunotherapy.

Breast cancers can be classified by their receptor status. Breast cancer cells may or may not have many different types of receptors, the three most important in the present classification being: estrogen receptor (ER), progesterone receptor (PR), and HER2/neu. Cells with or without these receptors are called ER positive (ER+), ER negative (ER-), PR positive (PR+), PR negative (PR-), HER2 positive (HER2+), and HER2 negative (HER2-). Cells with none of these receptors are called basal-like or triple negative. According to the invention, breast cancer can be categorized according to the receptor status as follows:
- HER2 positive breast cancer
- Triple negative breast cancer (TNBC): ER-, PR- and HER2-
- Luminal breast cancer: ER+ and HER2- (and preferably PR+).

According to the invention, the term "breast cancer" may include one or more of HER2 positive breast cancer, triple negative breast cancer and luminal breast cancer. According to the invention, in particular if prognostic aspects of the invention are concerned, the term "breast cancer" preferably relates to HER2 positive breast cancer and/or triple negative breast cancer, preferably HER2 positive breast cancer. According to the invention, in particular if aspects of measuring the eligibility of patients for antibody therapy are concerned, the term "breast cancer" preferably relates to HER2 positive breast cancer. According to the invention, in particular if aspects of measuring the eligibility of patients for chemotherapy are concerned, the term "breast cancer" preferably relates to luminal breast cancer and/or triple negative breast cancer.

ER+ cancer cells depend on estrogen for their growth, so they can be treated with drugs to block estrogen effects (e.g. tamoxifen), and generally have a better prognosis. HER2+ breast cancer had a worse prognosis. HER2+ cancer cells may respond to drugs such as the monoclonal antibody trastuzumab or the toxin labeled trastutumab emtasine (in combination with conventional chemotherapy), and this has improved the prognosis significantly.

The three main groups of medications used for adjuvant breast cancer treatment are hormone blocking therapy, chemotherapy, and monoclonal antibodies.

### Hormone blocking therapy

Some breast cancers require estrogen to continue growing. They can be identified by the presence of estrogen receptors (ER+) and progesterone receptors (PR+) on their surface (sometimes referred to together as hormone receptors). These ER+ cancers can be treated with drugs that either block the receptors, e.g. tamoxifen (Nolvadex), or alternatively block the production of estrogen with an aromatase inhibitor, e.g. anastrozole (Arimidex) or letrozole (Femara). Aromatase inhibitors, however, are only suitable for post-menopausal patients. This is because the active aromatase in postmenopausal women is different from the prevalent form in premenopausal women, and therefore these agents are ineffective in inhibiting the predominant aromatase of premenopausal women.

### Chemotherapy

Chemotherapy is predominately used for stage 2-4 disease and is particularly beneficial in estrogen receptor-negative (ER-) disease. They are given in combinations, usually for 3-6 months. One of the most common treatments is cyclophosphamide plus doxorubicin (adriamycin), known as AC. Most chemotherapy medications work by destroying fast-growing and/or fast-replicating cancer cells either by causing DNA damage upon replication or other mechanisms; these drugs also damage fast-growing normal cells where they cause serious side effects. Damage to the heart muscle is the most dangerous complication of doxorubicin. Sometimes a taxane drug, such as docetaxel, is added, and the regime is then known as CAT; taxane attacks the microtubules in cancer cells. Another common treatment, which produces equivalent results, is cyclophosphamide, methotrexate, and fluorouracil (CMF). (Chemotherapy can literally refer to any drug, but it is usually used to refer to traditional non-hormone treatments for cancer.)

### Monoclonal antibodies

Trastuzumab (Herceptin), a monoclonal antibody to HER2, is only effective in patients with HER2 amplification/overexpression. Trastuzumab, however, is expensive, and approximately 2% of patients suffer significant heart damage. Other monoclonal antibodies are also undergoing clinical trials. This includes anti HER2 antibodies conjugated to toxic moieties such as "T-DM1" or dierected against dimerization domains of HER2 such as "pertuzumab". Between 25 and thirty percent of breast cancers have an amplification of the HER2 gene or overexpression of its protein product. Overexpression of this receptor in breast cancer is associated with increased disease recurrence and worse prognosis.

According to the invention, a "carcinoma" is a malignant tumor derived from epithelial cells. This group represents the most common cancers, including the common forms of breast, prostate, lung and colon cancer.

"Adenocarcinoma" is a cancer that originates in glandular tissue. This tissue is also part of a larger tissue category known as epithelial tissue. Epithelial tissue includes skin, glands and a variety of other tissue that lines the cavities and organs of the body. Epithelium is derived embryologically from ectoderm, endoderm and mesoderm. To be classified as adenocarcinoma, the cells do not necessarily need to be part of a gland, as long as they have secretory properties. This form of carcinoma can occur in some higher mammals, including humans. Well differentiated adenocarcinomas tend to resemble the glandular tissue that they are derived from, while poorly differentiated may not. By staining the cells from a biopsy, a pathologist will determine whether the tumor is an adenocarcinoma or some other type of cancer. Adenocarcinomas can arise in many tissues of the body due to the ubiquitous nature of glands within the body.

While each gland may not be secreting the same substance, as long as there is an exocrine function to the cell, it is considered glandular and its malignant form is therefore named adenocarcinoma. Malignant adenocarcinomas invade other tissues and often metastasize given enough time to do so. Ovarian adenocarcinoma is the most common type of ovarian carcinoma. It includes the serous and mucinous adenocarcinomas, the clear cell adenocarcinoma and the endometrioid adenocarcinoma.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system. In one embodiment, the term "metastasis" according to the invention relates to lymph node metastasis.

A refractory cancer is a malignancy for which a particular treatment is ineffective, which is either initially unresponsive to treatment, or which becomes unresponsive over time. The terms "refractory", "unresponsive" or "resistant" are used interchangeably herein.

Preferably, a (therapeutic) treatment of cancer is selected from the group consisting of surgery, chemotherapy, radiation therapy and targeted therapy.

The term "surgery", as used herein, includes the removal of tumors in an operation. It is a common treatment for cancer. A surgeon may remove the tumors using local excision.

The term "chemotherapy", as used herein, refers to the use of chemotherapeutic agents or combinations of chemotherapeutic agents, preferably to stop the growth of cancer cells, either by killing the cells or by stopping them from dividing. When chemotherapy is taken by mouth or injected into a vein or muscle, the drugs enter the bloodstream and can reach cancer cells throughout the body (systemic chemotherapy). When chemotherapy is placed directly into the cerebrospinal fluid, an organ, or a body cavity such as the abdomen, the drugs mainly affect cancer cells in those areas (regional chemotherapy).

Chemotherapeutic agents according to the invention include cytostatic compounds and cytotoxic compounds. Traditional chemotherapeutic agents act by killing cells that divide rapidly, one of the main properties of most cancer cells. This means that chemotherapy also harms cells that divide rapidly under normal circumstances such as cells in the bone marrow, digestive tract, and hair follicles. This results in the most common side-effects of chemotherapy. According to the invention, the term "chemotherapy" preferably does not include antibodies that target proteins that are abnormally expressed in cancer cells (tumor antigens) and act through recruiting the patient's immune system to destroy tumor cells. Antibodies that target proteins that are abnormally expressed in cancer cells (tumor antigens) and act through a therapeutic moiety or agent conjugated to the antibody, however, can be viewed as a form of chemotherapy. However, in the strictest sense, the term "chemotherapy" according to the invention does not include targeted therapy.

According to the invention, the term "chemotherapeutic agent" includes taxanes, platinum compounds, nucleoside analogs, camptothecin analogs, anthracyclines, etoposide, bleomycin, vinorelbine, cyclophosphamide, and combinations thereof. According to the invention a reference to a chemotherapeutic agent is to include any prodrug such as ester, salt or derivative such as conjugate of said agent. Examples are conjugates of said agent with a carrier substance, e.g. protein-bound paclitaxel such as albumin-bound paclitaxel. Preferably, salts of said agent are pharmaceutically acceptable.

Taxanes are a class of diterpene compounds that were first derived from natural sources such as plants of the genus Taxus, but some have been synthesized artificially. The principal mechanism of action of the taxane class of drugs is the disruption of microtubule function, thereby inhibiting the process of cell division. Taxanes include docetaxel (Taxotere) and paclitaxel (Taxol).

According to the invention, the term "docetaxel" refers to a compound having the following formula:

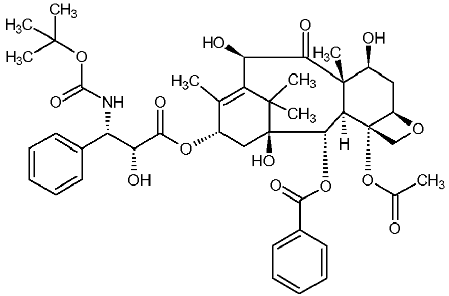

In particular, the term "docetaxel" refers to the compound 1,7β,10β-trihydroxy-9-oxo-5β,20-epoxytax-11-ene-2a,4,13a-triyl 4-acetate 2-benzoate 13-{(2R,3S)-3-[(tert-butoxycarbonyl)-amino]-2-hydroxy-3-phenylpropanoate}.

According to the invention, the term "paclitaxel" refers to a compound having the following formula:

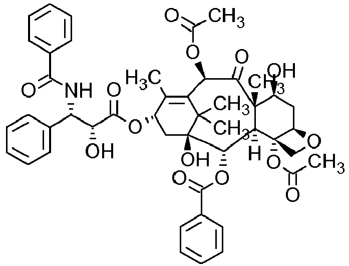

In particular, the term "paclitaxel" refers to the compound (2α,4α,5β,7β,10β,13α)-4,10-bis-(acetyloxy)-13-{[(2R,3S)-3-(benzoylamino)-2-hydroxy-3-phenylpropanoyl]oxy}-1,7-dihydroxy-9-oxo-5,20-epoxytax-11-en-2-yl benzoate.

According to the invention, the term "platinum compound" refers to compounds containing platinum in their structure such as platinum complexes and includes compounds such as cisplatin, carboplatin and oxaliplatin.

The term "cisplatin" or "cisplatinum" refers to the compound *cis*-diamminedichloroplatinum(II) (CDDP) of the following formula:

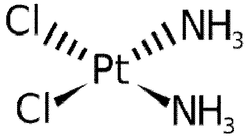

The term "carboplatin" refers to the compound cis-diammine(1,1-cyclobutanedicarboxylato)platinum(II) of the following formula:

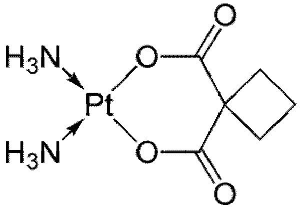

The term "oxaliplatin" refers to a compound which is a platinum compound that is complexed to a diaminocyclohexane carrier ligand of the following formula:

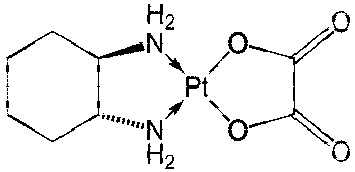

In particular, the term "oxaliplatin" refers to the compound [(1R,2R)-cyclohexane-1,2-diamine](ethanedioato-O,O')platinum(II). Oxaliplatin for injection is also marketed under the trade name Eloxatine.

The term "nucleoside analog" refers to a structural analog of a nucleoside, a category that includes both purine analogs and pyrimidine analogs.

The term "gemcitabine" is a compound which is a nucleoside analog of the following formula:

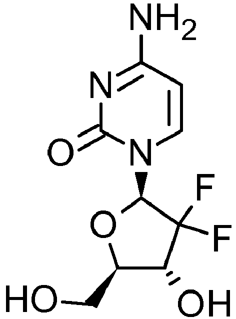

In particular, the term refers to the compound 4-amino-1-(2-deoxy-2,2-difluoro-β-D-erythro-pentofuranosyl)pyrimidin-2(1 H)-one or 4-amino-1-[(2R,4R,5R)-3,3-difluoro-4-hydroxy-5-(hydroxymethyl)oxolan-2-yl]-1,2-dihydropyrimidin-2-one.

The term "nucleoside analog" includes fluoropyrimidine derivatives such as fluorouracil and prodrugs thereof. The term "fluorouracil" or "5-fluorouracil" (5-FU or f5U) (sold under the brand names Adrucil, Carac, Efudix, Efudex and Fluoroplex) is a compound which is a pyrimidine analog of the following formula:

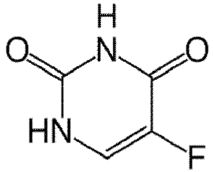

In particular, the term refers to the compound 5-fluoro-1 H-pyrimidine-2,4-dione.

The term "capecitabine" (Xeloda, Roche) refers to a chemotherapeutic agent that is a prodrug that is converted into 5-FU in the tissues. Capecitabine which may be orally administered has the following formula:

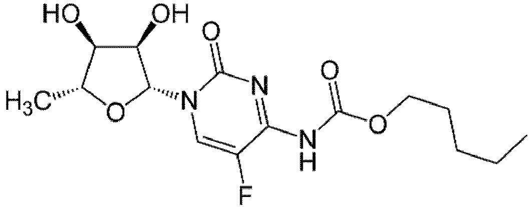

In particular, the term refers to the compound pentyl [1-(3,4-dihydroxy-5-methyltetrahydrofuran-2-yl)-5-fluoro-2-oxo-1 H-pyrimidin-4-yl]carbamate.

The term "folinic acid" or "leucovorin" refers to a compound useful in synergistic combination with the chemotherapy agent 5-fluorouracil. Thus, if reference is made herein to the administration of 5-fluorouracil or a prodrug thereof, said administration in one embodiment may comprise an administration in conjunction with folinic acid. Folinic acid has the following formula:

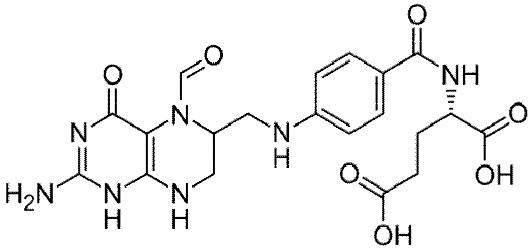

In particular, the term refers to the compound (2S)-2-{[4-[(2-amino-5-formyl-4-oxo-5,6,7,8-tetrahydro-1H-pteridin-6-yl)methylamino]benzoyl]amino}pentanedioic acid.

According to the invention, the term "camptothecin analog" refers to derivatives of the compound camptothecin (CPT; (S)-4-ethyl-4-hydroxy-1H-pyrano[3',4':6,7]indolizino[1,2-b] quinoline-3,14-(4H,12H)-dione). Preferably, the term "camptothecin analog" refers to compounds comprising the following structure:

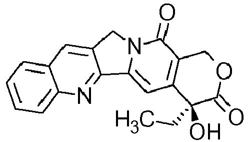

According to the invention, preferred camptothecin analogs are inhibitors of DNA enzyme topoisomerase I (topo I). Preferred camptothecin analogs according to the invention are irinotecan and topotecan.

Irinotecan is a drug preventing DNA from unwinding by inhibition of topoisomerase I. In chemical terms, it is a semisynthetic analogue of the natural alkaloid camptothecin having the following formula:

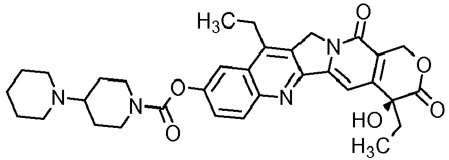

In particular, the term "irinotecan" refers to the compound (S)-4,11-diethyl-3,4,12,14-tetrahydro-4-hydroxy-3,14-dioxo1H-pyrano[3',4':6,7]-indolizino[1,2-b]quinolin-9-yl-[1,4'-bipiperidine]-1'-carboxylate.

Topotecan is a topoisomerase inhibitor of the formula:

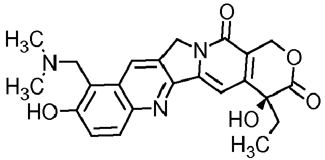

In particular, the term "topotecan" refers to the compound (S)-10-[(dimethylamino)methyl]-4-ethyl-4,9-dihydroxy-1H-pyrano[3',4':6,7]indolizino[1,2-b]quinoline-3,14(4H,12H)-dione monohydrochloride.

Anthracyclines are a class of drugs commonly used in cancer chemotherapy that are also antibiotics. Structurally, all anthracyclines share a common four-ringed 7,8,9,10-tetrahydrotetracene-5,12-quinone structure and usually require glycosylation at specific sites.

Anthracyclines preferably bring about one or more of the following mechanisms of action: 1. Inhibiting DNA and RNA synthesis by intercalating between base pairs of the DNA/RNA strand, thus preventing the replication of rapidly-growing cancer cells. 2. Inhibiting topoisomerase II enzyme, preventing the relaxing of supercoiled DNA and thus blocking DNA transcription and replication. 3. Creating iron-mediated free oxygen radicals that damage the DNA and cell membranes.

According to the invention, the term "anthracycline" preferably relates to an agent, preferably an anticancer agent for inducing apoptosis, preferably by inhibiting the rebinding of DNA in topoisomerase II.

Examples of anthracyclines and anthracycline analogs include, but are not limited to, daunorubicin (daunomycin), doxorubicin (adriamycin), epirubicin, idarubicin, rhodomycin, pyrarubicin, valrubicin, N-trifluoro-acetyl doxorubicin-14-valerate, aclacinomycin, morpholinodoxorubicin (morpholino-DOX), cyanomorpholino-doxorubicin (cyano-morpholino-DOX), 2-pyrrolino-doxorubicin (2-PDOX), 5-iminodaunomycin, mitoxantrone and aclacinomycin A (aclarubicin). Mitoxantrone is a member of the anthracendione class of compounds, which are anthracycline analogs that lack the sugar moiety of the anthracyclines but retain the planar polycylic aromatic ring structure that permits intercalation into DNA.

Specifically contemplated as anthracycline in the context of the present invention is epirubicin. Epirubicin is an anthracycline drug which has the following formula:

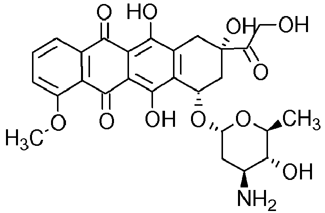

and is marketed under the trade name Ellence in the US and Pharmorubicin or Epirubicin Ebewe elsewhere. In particular, the term "epirubicin" refers to the compound (8R,10S)-10-[(2S,4S,5R,6S)-4-amino-5-hydroxy-6-methyl-oxan-2-yl]oxy-6,11-dihydroxy-8-(2-hydroxyacetyl)-1-methoxy-8-methyl-9,10-dihydro-7H-tetracen-5,12-dion. Epirubicin is favoured over doxorubicin, the most popular anthracycline, in some chemotherapy regimens as it appears to cause fewer side-effects.

The term "etoposide" refers to a semisynthetic derivative of podophyllotoxin that exhibits antitumor activity. Etoposide inhibits DNA synthesis by forming a complex with topoisomerase II and DNA. This complex induces breaks in double stranded DNA and prevents repair by topoisomerase II binding. Accumulated breaks in DNA prevent entry into the mitotic phase of cell division, and lead to cell death. Etoposide has the following formula:

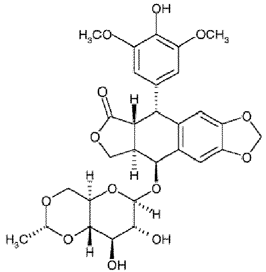

In particular, the term refers to the compound 4'-demethyl-epipodophyllotoxin 9-[4,6-O-(R)-ethylidene-beta-D-glucopyranoside], 4' -(dihydrogen phosphate).

The term "bleomycin" refers to a glycopeptide antibiotic produced by the bacterium Streptomyces verticillus. When used as an anticancer agent, it works by causing breaks in DNA. Bleomycin preferably comprises a compound having the following formula:

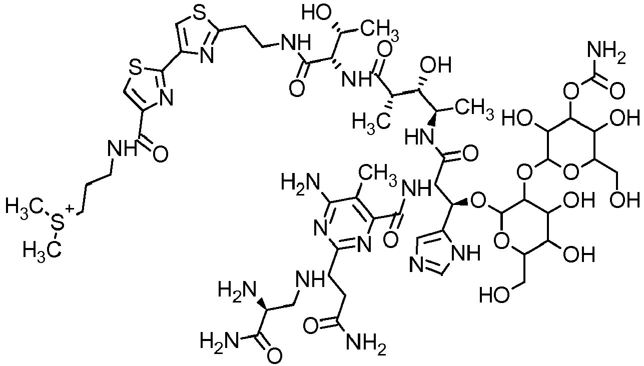

The term "vinorelbine" refers to an anti-mitotic chemotherapy drug that is a semi-synthetic vinca alkaloid and is given as a treatment for some types of cancer, including breast cancer and non-small cell lung cancer. Vinorelbine preferably comprises a compound having the following formula:

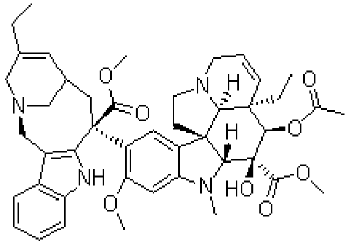

Cyclophosphamide is a nitrogen mustard alkylating agent from the oxazophorines group. The main use of cyclophosphamide is with other chemotherapy agents in the treatment of some forms of cancer. Cyclophosphamide preferably comprises a compound having the following formula:

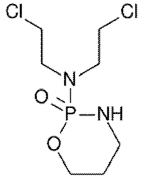

In the context of the present invention, the term "radiation therapy" refers to the use of high-energy x-rays or other types of radiation to kill cancer cells or keep them from growing. There are two types of radiation therapy. External radiation therapy uses a machine outside the body to send radiation toward the cancer. Internal radiation therapy uses a radioactive substance sealed in needles, seeds, wires, or catheters that are placed directly into or near the cancer. The way the radiation therapy is given depends on the type and stage of the cancer being treated.

According to the invention, the term "targeted therapy" relates to any therapy that can be used to target preferentially diseased cells such as cancer cells while non-diseased cells are not targeted or targeted to a lesser extent. Targeting of diseased cells preferably results in killing and/or impairment of proliferation or viability of diseased cells. Such therapy includes i) antibodies, antibody fragments, and proteins that are either naked or conjugated to a therapeutic moiety that target certain cell surface targets on diseased cells, for example, HER2/neu, (e.g. antibodies or antibody conjugates against HER2/neu as described above) or ii) small molecules which impair proliferation or viability of diseased cells. In a specific embodiment, the agent binds to an antigen that is expressed at a greater level on diseased than on normal stem cells. In a specific embodiment, the agent binds specifically to a tumor antigen. Traditional chemotherapy or radiotherapy is not considered a "targeted therapy" despite its often being aimed at the tumours. Furthermore, the term "antibody therapy" according to the invention preferably does not include therapy with antibodies, fragments or derivatives thereof that are conjugated to a therapeutic moiety but merely relates to therapy with antibodies, fragments or derivatives thereof acting through recruiting the patient's immune system to destroy tumor cells.

The term "antigen" relates to an agent such as a protein or peptide comprising an epitope against which an immune response is directed and/or is to be directed. In a preferred embodiment, an antigen is a tumor-associated antigen or tumor antigen, such as HER2/neu, i.e., a constituent of cancer cells which may be derived from the cytoplasm, the cell surface and the cell nucleus, in particular those antigens which are produced, preferably in large quantity, intracellular or as surface antigens on cancer cells.

In the context of the present invention, the term "tumor-associated antigen" or "tumor antigen" preferably relates to proteins that are under normal conditions specifically expressed in a limited number of tissues and/or organs or in specific developmental stages and are expressed or aberrantly expressed in one or more tumor or cancer tissues. In the context of the present invention, a tumor antigen is preferably associated with the cell surface of a cancer cell and is preferably not, only rarely or at a lower level expressed in normal tissues and cells. Preferably, according to the invention, a tumor antigen is not expressed in a cell if the level of expression is below the detection limit and/or if the level of expression is too low to allow binding by tumor antigen-specific antibodies added to the cells. A particularly preferred tumor antigen according to the invention is HER2/neu.

The term "HER2/neu" or simply "HER2" means "human epidermal growth factor receptor 2" and relates to a gene that is overexpressed due to gene amplification in approximately 25% of breast cancer patients and to the encoded gene product. HER2 is a member of the epidermal growth factor receptor (EGFR/ErbB) family. The term preferably relates, in particular, to a protein comprising, preferably consisting of the amino acid sequence of SEQ ID NO: 3 or 4 of the sequence listing or a variant of said amino acid sequence.

According to the invention, the term "tumor antigen-positive cancer" or "tumor antigen-positive tumor" or similar terms means a cancer or tumor involving cancer or tumor cells expressing a tumor antigen, preferably on the surface of said cancer cells or tumor cells. A tumor antigen is expressed on the surface of cells if it is located at the surface of said cells and is accessible to binding by tumor antigen-specific antibodies added to the cells.

The term "epitope" refers to an antigenic determinant in a molecule, i.e., to the part in a molecule that is recognized by the immune system, for example, that is recognized by an antibody. For example, epitopes are the discrete, three-dimensional sites on an antigen, which are recognized by the immune system. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents. An epitope of a protein preferably comprises a continuous or discontinuous portion of said protein and is preferably between 5 and 100, preferably between 5 and 50, more preferably between 8 and 30, most preferably between 10 and 25 amino acids in length, for example, the epitope may be preferably 8, 9, 10, 11, 12, 13, 14, 15, 16, 17, 18, 19, 20, 21, 22, 23, 24, or 25 amino acids in length.

The term "antibody" includes a glycoprotein comprising at least two heavy (H) chains and two light (L) chains inter-connected by disulfide bonds, and any molecule comprising an antigen-binding portion of such glycoprotein. The term "antibody" includes monoclonal antibodies, recombinant antibodies, human antibodies, humanized antibodies, chimeric antibodies, fragments or derivatives of antibodies, including, without limitation, single chain antibodies, e.g., scFv's and antigen-binding antibody fragments such as Fab and Fab' fragments and also includes all recombinant forms of antibodies, e.g., antibodies expressed in prokaryotes, unglycosylated antibodies, and any antigen-binding antibody fragments and derivatives as described herein. Each heavy chain is comprised of a heavy chain variable region (abbreviated herein as VH) and a heavy chain constant region. Each light chain is comprised of a light chain variable region (abbreviated herein as VL) and a light chain constant region. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "monoclonal antibody" as used herein refers to a preparation of antibody molecules of single molecular composition. A monoclonal antibody displays a single binding specificity and affinity. In one embodiment, the monoclonal antibodies are produced by a hybridoma which includes a B cell obtained from a non-human animal, e.g., mouse, fused to an immortalized cell.

The term "recombinant antibody", as used herein, includes all antibodies that are prepared, expressed, created or isolated by recombinant means, such as (a) antibodies isolated from an animal (e.g., a mouse) that is transgenic or transchromosomal with respect to the immunoglobulin genes or a hybridoma prepared therefrom, (b) antibodies isolated from a host cell transformed to express the antibody, e.g., from a transfectoma, (c) antibodies isolated from a recombinant, combinatorial antibody library, and (d) antibodies prepared, expressed, created or isolated by any other means that involve splicing of immunoglobulin gene sequences to other DNA sequences.

The term "human antibody", as used herein, is intended to include antibodies having variable and constant regions derived from human germline immunoglobulin sequences. Human antibodies may include amino acid residues not encoded by human germline immunoglobulin sequences (e.g., mutations introduced by random or site-specific mutagenesis *in vitro* or by somatic mutation *in vivo*).

The term "humanized antibody" refers to a molecule having an antigen binding site that is substantially derived from an immunoglobulin from a non-human species, wherein the remaining immunoglobulin structure of the molecule is based upon the structure and/or sequence of a human immunoglobulin. The antigen binding site may either comprise complete variable domains fused onto constant domains or only the complementarity determining regions (CDR) grafted onto appropriate framework regions in the variable domains. Antigen binding sites may be wild-type or modified by one or more amino acid substitutions, e.g. modified to resemble human immunoglobulins more closely. Some forms of humanized antibodies preserve all CDR sequences (for example a humanized mouse antibody which contains all six CDRs from the mouse antibody). Other forms have one or more CDRs which are altered with respect to the original antibody.

The term "chimeric antibody" refers to those antibodies wherein one portion of each of the amino acid sequences of heavy and light chains is homologous to corresponding sequences in antibodies derived from a particular species or belonging to a particular class, while the remaining segment of the chain is homologous to corresponding sequences in another. Typically the variable region of both light and heavy chains mimics the variable regions of antibodies derived from one species of mammals, while the constant portions are homologous to sequences of antibodies derived from another. One clear advantage to such chimeric forms is that the variable region can conveniently be derived from presently known sources using readily available B-cells or hybridomas from non-human host organisms in combination with constant regions derived from, for example, human cell preparations. While the variable region has the advantage of ease of preparation and the specificity is not affected by the source, the constant region being human, is less likely to elicit an immune response from a human subject when the antibodies are injected than would the constant region from a non human source. However the definition is not limited to this particular example.

Antibodies may be derived from different species, including but not limited to mouse, rat, rabbit, guinea pig and human.

Antibodies described herein include IgA such as IgA1 or IgA2, IgG1, IgG2, IgG3, IgG4, IgE, IgM, and IgD antibodies. In various embodiments, the antibody is an IgG1 antibody, more particularly an IgG1, kappa or IgG1, lambda isotype (i.e. IgG1, κ, λ), an IgG2a antibody (e.g. IgG2a, κ, λ), an IgG2b antibody (e.g. IgG2b, κ, λ), an IgG3 antibody (e.g. IgG3, κ, z) or an IgG4 antibody (e.g. IgG4, κ, λ).

As used herein, a "heterologous antibody" is defined in relation to a transgenic organism producing such an antibody. This term refers to an antibody having an amino acid sequence or an encoding nucleic acid sequence corresponding to that found in an organism not consisting of the transgenic organism, and being generally derived from a species other than the transgenic organism.

As used herein, a "heterohybrid antibody" refers to an antibody having light and heavy chains of different organismal origins. For example, an antibody having a human heavy chain associated with a murine light chain is a heterohybrid antibody.

The antibodies described herein are preferably isolated. An "isolated antibody" as used herein, is intended to refer to an antibody which is substantially free of other antibodies having different antigenic specificities (e.g., an isolated antibody that specifically binds to a tumor antigen is substantially free of antibodies that specifically bind antigens other than the tumor antigen). An isolated antibody that specifically binds to an epitope, isoform or variant of a human tumor antigen may, however, have cross-reactivity to other related antigens, e.g., from other species (e.g., tumor antigen species homologs). Moreover, an isolated antibody may be substantially free of other cellular material and/or chemicals. In one embodiment of the invention, a combination of "isolated" monoclonal antibodies relates to antibodies having different specificities and being combined in a well defined composition or mixture.

The terms "antigen-binding portion" of an antibody (or simply "binding portion") or "antigen-binding fragment" of an antibody (or simply "binding fragment") or similar terms refer to one or more fragments of an antibody that retain the ability to specifically bind to an antigen. It has been shown that the antigen-binding function of an antibody can be performed by fragments of a full-length antibody. Examples of binding fragments encompassed within the term "antigen-binding portion" of an antibody include (i) Fab fragments, monovalent fragments consisting of the VL, VH, CL and CH domains; (ii) F(ab')₂ fragments, bivalent fragments comprising two Fab fragments linked by a disulfide bridge at the hinge region; (iii) Fd fragments consisting of the VH and CH domains; (iv) Fv fragments consisting of the VL and VH domains of a single arm of an antibody, (v) dAb fragments (Ward et al., (1989) Nature 341: 544-546), which consist of a VH domain; (vi) isolated complementarity determining regions (CDR), and (vii) combinations of two or more isolated CDRs which may optionally be joined by a synthetic linker. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242: 423-426; and Huston et al. (1988) Proc. Natl. Acad. Sci. USA 85: 5879-5883). Such single chain antibodies are also intended to be encompassed within the term "antigen-binding fragment" of an antibody. A further example is binding-domain immunoglobulin fusion proteins comprising (i) a binding domain polypeptide that is fused to an immunoglobulin hinge region polypeptide, (ii) an immunoglobulin heavy chain CH2 constant region fused to the hinge region, and (iii) an immunoglobulin heavy chain CH3 constant region fused to the CH2 constant region. The binding domain polypeptide can be a heavy chain variable region or a light chain variable region. The binding-domain immunoglobulin fusion proteins are further disclosed in US 2003/0118592 and US 2003/0133939. These antibody fragments are obtained using conventional techniques known to those with skill in the art, and the fragments are screened for utility in the same manner as are intact antibodies.

The term "binding domain" characterizes in connection with the present invention a structure, e.g. of an antibody, which binds to/interacts with a given target structure/antigen/epitope. Thus, the binding domain according to the invention designates an "antigen-interaction-site".

All antibodies and derivatives of antibodies such as antibody fragments as described herein for the purposes of the invention are encompassed by the term "antibody". The term "antibody derivatives" refers to any modified form of an antibody, e.g., a conjugate of the antibody and another agent or antibody, or an antibody fragment.

Naturally occurring antibodies are generally monospecific, i.e. they bind to a single antigen. The present invention comprises antibodies binding to a target cell (by engaging a tumor antigen) and a second entity such as a cytotoxic cell (e.g. by engaging the CD3 receptor). The antibodies of the present invention may be bispecific or multispecific such as trispecific, tetraspecific and so on.

The term "bispecific molecule" is intended to include an agent which has two different binding specificities. For example, the molecule may bind to, or interact with (a) a cell surface antigen, and (b) a receptor such as an Fc receptor on the surface of an effector cell. The term "multispecific molecule" is intended to include an agent which has more than two different binding specificities. For example, the molecule may bind to, or interact with (a) a cell surface antigen, (b) a receptor such as an Fc receptor on the surface of an effector cell, and (c) at least one other component. Accordingly, the term "antibody against a tumor antigen" includes, but is not limited to, bispecific, trispecific, tetraspecific, and other multispecific molecules which are directed to a tumor antigen, and to other targets, such as Fc receptors on effector cells. The term "bispecific antibodies" also includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen binding sites (see e.g. , Holliger, P., et al. (1993) Proc. Natl. Acad. Sci. USA 90: 6444-6448; Poljak, R. J., et al. (1994) Structure 2: 1121-1123).

According to the invention, an antibody may exert its therapeutic effect through recruiting the patient's immune system to destroy tumor cells and/or through a therapeutic moiety or agent coupled to the antibody. For the purpose of the present invention, such antibody conjugates may be considered being encompassed by the term "chemotherapeutic agent" while antibodies exerting their therapeutic effect through recruiting the patient's immune system to destroy tumor cells are not.

In the context of the present invention, an antibody preferably is capable of acting through recruiting the patient's immune system to destroy tumor cells, i.e. the antibody, in particular when bound to its target such as a tumor antigen on a diseased cell, elicits immune effector functions as described herein. Preferably, said immune effector functions are directed against cells such as cancer cells carrying the tumor antigen HER2/neu on their surface.

The term "immune effector functions" in the context of the present invention includes any functions mediated by components of the immune system that result e.g. in the inhibition of tumor growth and/or inhibition of tumor development, including inhibition of tumor dissemination and metastasis. Preferably, immune effector functions result in killing of cancer cells. Such functions comprise complement dependent cytotoxicity (CDC), antibody-dependent cell-mediated cytotoxicity (ADCC), antibody-dependent cell-mediated phagocytosis (ADCP), induction of apoptosis in the cells carrying the tumor-associated antigen, cytolysis of the cells carrying the tumor-associated antigen, and/or inhibition of proliferation of the cells carrying the tumor-associated antigen. Binding agents may also exert an effect simply by binding to tumor-associated antigens on the surface of a cancer cell. For example, antibodies may block the function of the tumor-associated antigen or induce apoptosis just by binding to the tumor-associated antigen on the surface of a cancer cell.

### Antibody-dependent cell-mediated cytotoxicity

ADCC describes the cell-killing ability of effector cells, in particular lymphocytes, which preferably requires the target cell being marked by an antibody.

ADCC preferably occurs when antibodies bind to antigens on tumor cells and the antibody Fc domains engage Fc receptors (FcR) on the surface of immune effector cells. Several families of Fc receptors have been identified, and specific cell populations characteristically express defined Fc receptors. ADCC can be viewed as a mechanism to directly induce a variable degree of immediate tumor destruction that leads to antigen presentation and the induction of tumor-directed T-cell responses. Preferably, *in vivo* induction of ADCC will lead to tumor-directed T-cell responses and host-derived antibody responses.

### Complement-dependent cytotoxicity

CDC is another cell-killing method that can be directed by antibodies. IgM is the most effective isotype for complement activation. IgG1 and IgG3 are also both very effective at directing CDC via the classical complement-activation pathway. Preferably, in this cascade, the formation of antigen-antibody complexes results in the uncloaking of multiple C1q binding sites in close proximity on the C_{H}2 domains of participating antibody molecules such as IgG molecules (C1q is one of three subcomponents of complement C1). Preferably these uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. Preferably, the complement cascade ends in the formation of a membrane attack complex, which creates pores in the cell membrane that facilitate free passage of water and solutes into and out of the cell.

In order to inhibit tumor growth and/or tumor development, according to the invention, an antibody may be conjugated to a therapeutic moiety or agent, such as a cytotoxin, a drug (e.g., an immunosuppressant) or a radioisotope. A cytotoxin or cytotoxic agent includes any agent that is detrimental to and, in particular, kills cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, amanitin, 1-dehydrotestosterone, glucocorticoids, procaine, tetracaine, lidocaine, propranolol, and puromycin and analogs or homologs thereof. Suitable therapeutic agents for forming antibody conjugates include, but are not limited to, antimetabolites (e.g., methotrexate, 6-mercaptopurine, 6-thioguanine, cytarabine, fludarabin, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC), and anti-mitotic agents (e.g., vincristine and vinblastine). In a preferred embodiment, the therapeutic agent is a cytotoxic agent or a radiotoxic agent. In another embodiment, the therapeutic agent is an immunosuppressant. In yet another embodiment, the therapeutic agent is GM-CSF. In a preferred embodiment, the therapeutic agent is doxorubicin, cisplatin, bleomycin, sulfate, carmustine, chlorambucil, cyclophosphamide or ricin A.

Antibodies also can be conjugated to a radioisotope, e.g., iodine-131, yttrium-90 or indium-111, to generate cytotoxic radiopharmaceuticals.

A particular conjugated antibody is trastuzumab emtansine which is an antibody-drug conjugate consisting of the monoclonal antibody trastuzumab (Herceptin) linked to the cytotoxic agent mertansine (DM1). Each molecule of trastuzumab emtansine consists of a single trastuzumab molecule bound to several molecules of mertansine, a cytotoxic maytansinoid containing a sulfhydryl group, through a crosslinking reagent known as SMCC (succinimidyl trans-4-(maleimidylmethyl)cyclohexane-1-carboxylate). The fact that mertansine is only released after the antibody-drug conjugate has been taken up by a tumor cell reduces toxic effects while maintaining antitumor efficacy. Trastuzumab alone stops growth of cancer cells by binding to the HER2/neu receptor, whereas mertansine enters cells and destroys them by binding to tubulin. For the purpose of the present invention, trastuzumab emtansine may be considered being encompassed by the term "chemotherapeutic agent" since it does not exert its therapeutic effect through recruiting the patient's immune system to destroy tumor cells.

The antibody conjugates of the invention can be used to modify a given biological response, and the drug moiety is not to be construed as limited to classical chemical therapeutic agents. For example, the drug moiety may be a protein or polypeptide possessing a desired biological activity. Such proteins may include, for example, an enzymatically active toxin, or active fragment thereof, such as abrin, ricin A, pseudomonas exotoxin, or diphtheria toxin; a protein such as tumor necrosis factor or interferon-y; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known, see, e.g., Arnon et al., "Monoclonal Antibodies For Immunotargeting Of Drugs In Cancer Therapy", in Monoclonal Antibodies And Cancer Therapy, Reisfeld et al. (eds. ), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pincheraet al. (eds. ), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (eds.), pp. 303-16 (Academic Press 1985), and Thorpe et al., "The Preparation And Cytotoxic Properties Of Antibody-Toxin Conjugates", Immunol. Rev., 62: 119-58 (1982).

The term "antibody against a tumor antigen" or similar terms relates to an antibody directed to or having the ability of binding to the tumor antigen. The term "binding" according to the invention preferably relates to a specific binding.

According to the present invention, an antibody is capable of binding to a predetermined target if it has a significant affinity for said predetermined target and binds to said predetermined target in standard assays. "Affinity" or "binding affinity" is often measured by equilibrium dissociation constant (K_{D}). Preferably, the term "significant affinity" refers to the binding to a predetermined target with a dissociation constant (K_{D}) of 10⁻⁵ M or lower, 10⁻⁶ M or lower, 10⁻⁷ M or lower, 10⁻⁸ M or lower, 10⁻⁹ M or lower, 10⁻¹⁰ M or lower, 10⁻¹¹ M or lower, or 10⁻¹² M or lower.

An antibody is not (substantially) capable of binding to a target if it has no significant affinity for said target and does not bind significantly, in particular does not bind detectably, to said target in standard assays. Preferably, the antibody does not detectably bind to said target if present in a concentration of up to 2, preferably 10, more preferably 20, in particular 50 or 100 µg/ml or higher. Preferably, an antibody has no significant affinity for a target if it binds to said target with a K_{D} that is at least 10-fold, 100-fold, 10³-fold, 10⁴-fold, 10⁵-fold, or 10⁶-fold higher than the K_{D} for binding to the predetermined target to which the antibody is capable of binding. For example, if the K_{D} for binding of an antibody to the target to which the antibody is capable of binding is 10-⁷M, the K_{D} for binding to a target for which the antibody has no significant affinity would be is at least 10⁻⁶ M, 10⁻⁵ M, 10⁻⁴ M, 10⁻³ M, 10⁻² M, or 10⁻⁷ M.

An antibody is specific for a predetermined target if it is capable of binding to said predetermined target while it is not capable of binding to other targets, i.e. has no significant affinity for other targets and does not significantly bind to other targets in standard assays. According to the invention, an antibody is specific for a tumor antigen if it is capable of binding to the tumor antigen but is not (substantially) capable of binding to other targets. Preferably, an antibody is specific for a tumor antigen if the affinity for and the binding to such other targets does not significantly exceed the affinity for or binding to tumor antigen-unrelated proteins such as bovine serum albumin (BSA), casein, human serum albumin (HSA) or non-tumor antigen transmembrane proteins such as MHC molecules or transferrin receptor or any other specified polypeptide. Preferably, an antibody is specific for a predetermined target if it binds to said target with a K_{D} that is at least 10-fold, 100-fold, 10³-fold, 10⁴-fold, 10⁵-fold, or 10⁶-fold lower than the K_{D} for binding to a target for which it is not specific. For example, if the K_{D} for binding of an antibody to the target for which it is specific is 10⁻⁷ M, the K_{D} for binding to a target for which it is not specific would be at least 10⁻⁶ M, 10⁻⁵ M, 10⁻⁴ M, 10⁻³ M, 10⁻² M, or 10⁻⁷ M.

Binding of an antibody to a target can be determined experimentally using any suitable method; see, for example, Berzofsky et al., "Antibody-Antigen Interactions" In Fundamental Immunology, Paul, W. E., Ed., Raven Press New York, N Y (1984), Kuby, Janis Immunology, W. H. Freeman and Company New York, N Y (1992), and methods described herein. Affinities may be readily determined using conventional techniques, such as by equilibrium dialysis; by using the BIAcore 2000 instrument, using general procedures outlined by the manufacturer; by radioimmunoassay using radiolabeled target antigen; or by another method known to the skilled artisan. The affinity data may be analyzed, for example, by the method of Scatchard et al., Ann N.Y. Acad. ScL, 51:660 (1949). The measured affinity of a particular antibody-antigen interaction can vary if measured under different conditions, e.g., salt concentration, pH. Thus, measurements of affinity and other antigen-binding parameters, e.g., K_{D}, IC₅₀, are preferably made with standardized solutions of antibody and antigen, and a standardized buffer.

As used herein, "isotype" refers to the antibody class (e.g., IgM or IgG1) that is encoded by heavy chain constant region genes.

As used herein, "isotype switching" refers to the phenomenon by which the class, or isotype, of an antibody changes from one Ig class to one of the other Ig classes.

The term "naturally occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally occurring.

The term "rearranged" as used herein refers to a configuration of a heavy chain or light chain immunoglobulin locus wherein a V segment is positioned immediately adjacent to a D-J or J segment in a conformation encoding essentially a complete VH or VL domain, respectively. A rearranged immunoglobulin (antibody) gene locus can be identified by comparison to germline DNA; a rearranged locus will have at least one recombined heptamer/nonamer homology element.

The term "unrearranged" or "germline configuration" as used herein in reference to a V segment refers to the configuration wherein the V segment is not recombined so as to be immediately adjacent to a D or J segment.

Preferably, binding of an antibody against a tumor antigen to cells expressing the tumor antigen induces or mediates killing of cells expressing the tumor antigen. The cells expressing a tumor antigen are preferably cancer cells and are, in particular, cells of the cancer diseases described herein such as cancer cells of breast cancer. Preferably, the antibody induces or mediates killing of cells by inducing one or more of complement dependent cytotoxicity (CDC) mediated lysis, antibody dependent cellular cytotoxicity (ADCC) mediated lysis, apoptosis, and inhibition of proliferation of cells expressing a tumor antigen. Preferably, ADCC mediated lysis of cells takes place in the presence of effector cells, which in particular embodiments are selected from the group consisting of monocytes, mononuclear cells, NK cells and PMNs. Inhibiting proliferation of cells can be measured in vitro by determining proliferation of cells in an assay using bromodeoxyuridine (5-bromo-2-deoxyuridine, BrdU). BrdU is a synthetic nucleoside which is an analogue of thymidine and can be incorporated into the newly synthesized DNA of replicating cells (during the S phase of the cell cycle), substituting for thymidine during DNA replication. Detecting the incorporated chemical using, for example, antibodies specific for BrdU indicates cells that were actively replicating their DNA.

In preferred embodiments, antibodies described herein can be characterized by one or more of the following properties:
a) specificity for a tumor antigen;
b) a binding affinity to a tumor antigen of about 100 nM or less, preferably, about 5-10 nM or less and, more preferably, about 1-3 nM or less,
c) the ability to induce or mediate CDC on tumor antigen positive cells;
d) the ability to induce or mediate ADCC on tumor antigen positive cells;
e) the ability to inhibit the growth of tumor antigen positive cells;
f) the ability to induce apoptosis of tumor antigen positive cells.

In one embodiment, an antibody against a tumor antigen has the ability of binding to an epitope present in the tumor antigen, preferably an epitope located within the extracellular domains of the tumor antigen. Preferably, an antibody against a tumor antigen is specific for the tumor antigen. Preferably, an antibody against a tumor antigen binds to the tumor antigen expressed on the cell surface. In particular preferred embodiments, an antibody against a tumor antigen binds to native epitopes of the tumor antigen present on the surface of living cells.

It is to be understood that the antibodies described herein may be delivered to a patient by administering a nucleic acid such as RNA encoding the antibody and/or by administering a host cell comprising a nucleic acid such as RNA encoding the antibody. Thus, a nucleic acid encoding an antibody when administered to a patient may be present in naked form or in a suitable delivery vehicle such as in the form of liposomes or viral particles, or within a host cell. The nucleic acid provided can produce the antibody over extended time periods in a sustained manner mitigating the instability at least partially observed for therapeutic antibodies. Nucleic acids to be delivered to a patient can be produced by recombinant means. If a nucleic acid is administered to a patient without being present within a host cell, it is preferably taken up by cells of the patient for expression of the antibody encoded by the nucleic acid. If a nucleic acid is administered to a patient while being present within a host cell, it is preferably expressed by the host cell within the patient so as to produce the antibody encoded by the nucleic acid.

The term "nucleic acid", as used herein, is intended to include DNA and RNA such as genomic DNA, cDNA, mRNA, recombinantly produced and chemically synthesized molecules. A nucleic acid may be single-stranded or double-stranded. RNA includes in vitro transcribed RNA (IVT RNA) or synthetic RNA.

Nucleic acids may be comprised in a vector. The term "vector" as used herein includes any vectors known to the skilled person including plasmid vectors, cosmid vectors, phage vectors such as lambda phage, viral vectors such as adenoviral or baculoviral vectors, or artificial chromosome vectors such as bacterial artificial chromosomes (BAC), yeast artificial chromosomes (YAC), or P1 artificial chromosomes (PAC). Said vectors include expression as well as cloning vectors. Expression vectors comprise plasmids as well as viral vectors and generally contain a desired coding sequence and appropriate DNA sequences necessary for the expression of the operably linked coding sequence in a particular host organism (e.g., bacteria, yeast, plant, insect, or mammal) or in in vitro expression systems. Cloning vectors are generally used to engineer and amplify a certain desired DNA fragment and may lack functional sequences needed for expression of the desired DNA fragments.

In the context of the present invention, the term "RNA" relates to a molecule which comprises ribonucleotide residues and preferably being entirely or substantially composed of ribonucleotide residues. "Ribonucleotide" relates to a nucleotide with a hydroxyl group at the 2'-position of a β-D-ribofuranosyl group. The term includes double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

According to the present invention, the term "RNA" includes and preferably relates to "mRNA" which means "messenger RNA" and relates to a "transcript" which may be produced using DNA as template and encodes a peptide or protein. mRNA typically comprises a 5' non translated region (5'-UTR), a protein or peptide coding region and a 3' non translated region (3'-UTR). mRNA has a limited halftime in cells and in vitro. Preferably, mRNA is produced by in vitro transcription using a DNA template. In one embodiment of the invention, the RNA is obtained by in vitro transcription or chemical synthesis. The in vitro transcription methodology is known to the skilled person. For example, there is a variety of in vitro transcription kits commercially available.

In one embodiment of the present invention, RNA is self-replicating RNA, such as single stranded self-replicating RNA. In one embodiment, the self-replicating RNA is single stranded RNA of positive sense. In one embodiment, the self-replicating RNA is viral RNA or RNA derived from viral RNA. In one embodiment, the self-replicating RNA is alphaviral genomic RNA or is derived from alphaviral genomic RNA. In one embodiment, the self-replicating RNA is a viral gene expression vector. In one embodiment, the virus is Semliki forest virus. In one embodiment, the self-replicating RNA contains one or more transgenes at least one of said transgenes encoding the antibody described herein. In one embodiment, if the RNA is viral RNA or derived from viral RNA, the transgenes may partially or completely replace viral sequences such as viral sequences encoding structural proteins. In one embodiment, the self-replicating RNA is *in vitro* transcribed RNA.

In order to increase expression and/or stability of the RNA used according to the present invention, it may be modified, preferably without altering the sequence of the expressed peptide or protein.

The term "modification" in the context of RNA as used according to the present invention includes any modification of RNA which is not naturally present in said RNA.

In one embodiment of the invention, the RNA used according to the invention does not have uncapped 5'-triphosphates. Removal of such uncapped 5'-triphosphates can be achieved by treating RNA with a phosphatase.

The RNA according to the invention may have modified naturally occurring or synthetic ribonucleotides in order to increase its stability and/or decrease cytotoxicity. For example, in one embodiment, in the RNA used according to the invention 5-methylcytidine is substituted partially or completely, preferably completely, for cytidine. Alternatively or additionally, in one embodiment, in the RNA used according to the invention pseudouridine is substituted partially or completely, preferably completely, for uridine.

In one embodiment, the term "modification" relates to providing an RNA with a 5'-cap or 5'-cap analog. The term "5'-cap" refers to a cap structure found on the 5'-end of an mRNA molecule and generally consists of a guanosine nucleotide connected to the mRNA via an unusual 5' to 5' triphosphate linkage. In one embodiment, this guanosine is methylated at the 7-position. The term "conventional 5'-cap" refers to a naturally occurring RNA 5'-cap, preferably to the 7-methylguanosine cap (m7G). In the context of the present invention, the term "5'-cap" includes a 5'-cap analog that resembles the RNA cap structure and is modified to possess the ability to stabilize RNA if attached thereto, preferably in vivo and/or in a cell.

Providing an RNA with a 5'-cap or 5'-cap analog may be achieved by in vitro transcription of a

DNA template in the presence of said 5'-cap or 5'-cap analog, wherein said 5'-cap is cotranscriptionally incorporated into the generated RNA strand, or the RNA may be generated, for example, by in vitro transcription, and the 5'-cap may be attached to the RNA post-transcriptionally using capping enzymes, for example, capping enzymes of vaccinia virus.

The RNA may comprise further modifications. For example, a further modification of the RNA used in the present invention may be an extension or truncation of the naturally occurring poly(A) tail or an alteration of the 5'- or 3'-untranslated regions (UTR) such as introduction of a UTR which is not related to the coding region of said RNA, for example, the insertion of one or more, preferably two copies of a 3'-UTR derived from a globin gene, such as alpha2-globin, alpha1-globin, beta-globin, preferably beta-globin, more preferably human beta-globin.

Therefore, in order to increase stability and/or expression of the RNA used according to the present invention, it may be modified so as to be present in conjunction with a poly-A sequence, preferably having a length of 10 to 500, more preferably 30 to 300, even more preferably 65 to 200 and especially 100 to 150 adenosine residues. In an especially preferred embodiment the poly-A sequence has a length of approximately 120 adenosine residues. In addition, incorporation of two or more 3'-non translated regions (UTR) into the 3'-non translated region of an RNA molecule can result in an enhancement in translation efficiency. In one particular embodiment the 3'-UTR is derived from the human β-globin gene.

Preferably, RNA if delivered to, i.e. transfected into, a cell, in particular a cell present in vivo, expresses the protein or peptide it encodes.

The term "transfection" relates to the introduction of nucleic acids, in particular RNA, into a cell. For purposes of the present invention, the term "transfection" also includes the introduction of a nucleic acid into a cell or the uptake of a nucleic acid by such cell, wherein the cell may be present in a subject, e.g., a patient. Thus, according to the present invention, a cell for transfection of a nucleic acid described herein can be present *in vitro* or *in vivo,* e.g. the cell can form part of an organ, a tissue and/or an organism of a patient. According to the invention, transfection can be transient or stable. For some applications of transfection, it is sufficient if the transfected genetic material is only transiently expressed. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. RNA can be transfected into cells to transiently express its coded protein.

The term "stability" of RNA relates to the "half-life" of RNA. "Half-life" relates to the period of time which is needed to eliminate half of the activity, amount, or number of molecules. In the context of the present invention, the half-life of an RNA is indicative for the stability of said RNA. The half-life of RNA may influence the "duration of expression" of the RNA. It can be expected that RNA having a long half-life will be expressed for an extended time period.

In the context of the present invention, the term "transcription" relates to a process, wherein the genetic code in a DNA sequence is transcribed into RNA. Subsequently, the RNA may be translated into protein. According to the present invention, the term "transcription" comprises *"in vitro* transcription", wherein the term *"in vitro* transcription" relates to a process wherein RNA, in particular mRNA, is *in vitro* synthesized in a cell-free system, preferably using appropriate cell extracts. Preferably, cloning vectors are applied for the generation of transcripts. These cloning vectors are generally designated as transcription vectors and are according to the present invention encompassed by the term "vector".

The term "translation" according to the invention relates to the process in the ribosomes of a cell by which a strand of messenger RNA directs the assembly of a sequence of amino acids to make a peptide or protein.

The term "expression" is used according to the invention in its most general meaning and comprises the production of RNA and/or peptides or proteins, e.g. by transcription and/or translation. With respect to RNA, the term "expression" or "translation" relates in particular to the production of peptides or proteins. It also comprises partial expression of nucleic acids. Moreover, expression can be transient or stable. According to the invention, the term expression also includes an "aberrant expression" or "abnormal expression".

"Aberrant expression" or "abnormal expression" means according to the invention that expression is altered, preferably increased, compared to a reference, e.g. a state in a subject not having a disease associated with aberrant or abnormal expression of a certain protein, e.g., a tumor antigen. An increase in expression refers to an increase by at least 10%, in particular at least 20%, at least 50% or at least 100%, or more. In one embodiment, expression is only found in a diseased tissue, while expression in a healthy tissue is repressed.

According to the invention, the term "RNA encoding" means that RNA, if present in the appropriate environment, preferably within a cell, can be expressed to produce a protein or peptide it encodes.

Some aspects of the invention rely on the adoptive transfer of host cells which are transfected *in vitro* with a nucleic acid such as RNA encoding an antibody described herein and transferred to recipients such as patients, preferably after ex *vivo* expansion from low precursor frequencies to clinically relevant cell numbers. The host cells used for treatment according to the invention may be autologous, allogeneic, or syngeneic to a treated recipient.

The term "autologous" is used to describe anything that is derived from the same subject. For example, "autologous transplant" refers to a transplant of tissue or organs derived from the same subject. Such procedures are advantageous because they overcome the immunological barrier which otherwise results in rejection.

The term "allogeneic" is used to describe anything that is derived from different individuals of the same species. Two or more individuals are said to be allogeneic to one another when the genes at one or more loci are not identical.

The term "syngeneic" is used to describe anything that is derived from individuals or tissues having identical genotypes, i.e., identical twins or animals of the same inbred strain, or their tissues.

The term "heterologous" is used to describe something consisting of multiple different elements. As an example, the transfer of one individual's bone marrow into a different individual constitutes a heterologous transplant. A heterologous gene is a gene derived from a source other than the subject.

The term "peptide" according to the invention comprises oligo- and polypeptides and refers to substances comprising two or more, preferably 3 or more, preferably 4 or more, preferably 6 or more, preferably 8 or more, preferably 9 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" refers to large peptides, preferably to peptides with more than 100 amino acid residues, but in general the terms "peptides" and "proteins" are synonyms and are used interchangeably herein.

The teaching given herein with respect to specific amino acid sequences, e.g. those shown in the sequence listing, is to be construed so as to also relate to variants of said specific sequences resulting in sequences which are functionally equivalent to said specific sequences, e.g. amino acid sequences exhibiting properties identical or similar to those of the specific amino acid sequences.

The term "variant" according to the invention refers, in particular, to mutants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. A species homolog is a nucleic acid or amino acid sequence with a different species of origin from that of a given nucleic acid or amino acid sequence. The term "variant" shall encompass any posttranslationally modified variants and conformation variants.

For the purposes of the present invention, "variants" of an amino acid sequence comprise amino acid insertion variants, amino acid addition variants, amino acid deletion variants and/or amino acid substitution variants. Amino acid deletion variants that comprise the deletion at the N-terminal and/or C-terminal end of the protein are also called N-terminal and/or C-terminal truncation variants.

Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid addition variants comprise amino- and/or carboxy-terminal fusions of one or more amino acids, such as 1, 2, 3, 5, 10, 20, 30, 50, or more amino acids.

Amino acid deletion variants are characterized by the removal of one or more amino acids from the sequence, such as by removal of 1, 2, 3, 5, 10, 20, 30, 50, or more amino acids. The deletions may be in any position of the protein.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties. Preferably, amino acid changes in protein variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids.

Preferably the degree of similarity, preferably identity between a given amino acid sequence and an amino acid sequence which is a variant of said given amino acid sequence will be at least about 60%, 65%, 70%, 80%, 81%, 82%, 83%, 84%, 85%, 86%, 87%, 88%, 89%, 90%, 91%, 92%, 93%, 94%, 95%, 96%, 97%, 98%, or 99%. The degree of similarity or identity is given preferably for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given preferably for at least about 20, at least about 40, at least about 60, at least about 80, at least about 100, at least about 120, at least about 140, at least about 160, at least about 180, or about 200 amino acids, preferably continuous amino acids. In preferred embodiments, the degree of similarity or identity is given for the entire length of the reference amino acid sequence. The alignment for determining sequence similarity, preferably sequence identity can be done with art known tools, preferably using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences.

The term "percentage identity" is intended to denote a percentage of amino acid residues which are identical between the two sequences to be compared, obtained after the best alignment, this percentage being purely statistical and the differences between the two sequences being distributed randomly and over their entire length. Sequence comparisons between two amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

The term "cell" or "host cell" preferably relates to an intact cell, i.e. a cell with an intact membrane that has not released its normal intracellular components such as enzymes, organelles, or genetic material. An intact cell preferably is a viable cell, i.e. a living cell capable of carrying out its normal metabolic functions. Preferably said term relates according to the invention to any cell which can be transfected with an exogenous nucleic acid. Preferably, the cell when transfected with an exogenous nucleic acid and transferred to a recipient can express the nucleic acid in the recipient. The term "cell" includes bacterial cells; other useful cells are yeast cells, fungal cells or mammalian cells. Suitable bacterial cells include cells from gram-negative bacterial strains such as strains of Escherichia coli, Proteus, and Pseudomonas, and gram-positive bacterial strains such as strains of Bacillus, Streptomyces, Staphylococcus, and Lactococcus. Suitable fungal cell include cells from species of Trichoderma, Neurospora, and Aspergillus. Suitable yeast cells include cells from species of Saccharomyces (Tor example Saccharomyces cerevisiae), Schizosaccharomyces (for example Schizo saccharomyces pombe), Pichia (for example Pichia pastoris and Pichia methanolicd), and Hansenula. Suitable mammalian cells include for example CHO cells, BHK cells, HeLa cells, COS cells, 293 HEK and the like. However, amphibian cells, insect cells, plant cells, and any other cells used in the art for the expression of heterologous proteins can be used as well. Mammalian cells are particularly preferred for adoptive transfer, such as cells from humans, mice, hamsters, pigs, goats, and primates. The cells may be derived from a large number of tissue types and include primary cells and cell lines such as cells of the immune system, in particular antigen-presenting cells such as dendritic cells and T cells, stem cells such as hematopoietic stem cells and mesenchymal stem cells and other cell types. An antigen-presenting cell is a cell that displays antigen in the context of major histocompatibility complex on its surface. T cells may recognize this complex using their T cell receptor (TCR).

The term "transgenic animal" refers to an animal having a genome comprising one or more transgenes, preferably heavy and/or light chain transgenes, or transchromosomes (either integrated or non-integrated into the animal's natural genomic DNA) and which is preferably capable of expressing the transgenes. For example, a transgenic mouse can have a human light chain transgene and either a human heavy chain transgene or human heavy chain transchromosome, such that the mouse produces human anti-tumor antigen antibodies when immunized with a tumor antigen and/or cells expressing a tumor antigen. The human heavy chain transgene can be integrated into the chromosomal DNA of the mouse, as is the case for transgenic mice, e.g., HuMAb mice, such as HCo7 or HCol2 mice, or the human heavy chain transgene can be maintained extrachromosomally, as is the case for transchromosomal (e.g., KM) mice as described in WO 02/43478. Such transgenic and transchromosomal mice may be capable of producing multiple isotypes of human monoclonal antibodies to a tumor antigen (e.g., IgG, IgA and/or IgE) by undergoing V-D-J recombination and isotype switching.

"Reduce", "decrease" or "inhibit" as used herein means an overall decrease or the ability to cause an overall decrease, preferably of 5% or greater, 10% or greater, 20% or greater, more preferably of 50% or greater, and most preferably of 75% or greater, in the level, e.g. in the level of expression or in the level of proliferation of cells.

Terms such as "increase" or "enhance" preferably relate to an increase or enhancement by about at least 10%, preferably at least 20%, preferably at least 30%, more preferably at least 40%, more preferably at least 50%, even more preferably at least 80%, and most preferably at least 100%, at least 200%, at least 500%, at least 1000%, at least 10000% or even more.

Antibodies described herein can be produced by a variety of techniques, including conventional monoclonal antibody methodology, e.g., the standard somatic cell hybridization technique of Kohler and Milstein, Nature 256: 495 (1975). Although somatic cell hybridization procedures are preferred, in principle, other techniques for producing monoclonal antibodies can be employed, e.g., viral or oncogenic transformation of B-lymphocytes or phage display techniques using libraries of antibody genes.

The preferred animal system for preparing hybridomas that secrete monoclonal antibodies is the murine system. Hybridoma production in the mouse is a very well established procedure. Immunization protocols and techniques for isolation of immunized splenocytes for fusion are known in the art. Fusion partners (e.g., murine myeloma cells) and fusion procedures are also known.

Other preferred animal systems for preparing hybridomas that secrete monoclonal antibodies are the rat and the rabbit system (e.g. described in Spieker-Polet et al., Proc. Natl. Acad. Sci. U.S.A. 92:9348 (1995), see also Rossi et al., Am. J. Clin. Pathol. 124: 295 (2005)).

In yet another preferred embodiment, human monoclonal antibodies can be generated using transgenic or transchromosomal mice carrying parts of the human immune system rather than the mouse system. These transgenic and transchromosomic mice include mice known as Hu-MAb mice and KM mice, respectively, and are collectively referred to herein as "transgenic mice." The production of human antibodies in such transgenic mice can be performed as described in detail for CD20 in WO2004 035607.

Yet another strategy for generating monoclonal antibodies is to directly isolate genes encoding antibodies from lymphocytes producing antibodies of defined specificity e.g. see Babcock et al., 1996; A novel strategy for generating monoclonal antibodies from single, isolated lymphocytes producing antibodies of defined specificities. For details of recombinant antibody engineering see also Welschof and Kraus, Recombinant antibodes for cancer therapy ISBN-0-89603-918-8 and Benny K.C. Lo Antibody Engineering ISBN 1-58829-092-1.

To generate antibodies, mice can be immunized with carrier-conjugated peptides derived from the antigen sequence, i.e. the sequence against which the antibodies are to be directed, an enriched preparation of recombinantly expressed antigen or fragments thereof and/or cells expressing the antigen, as described. Alternatively, mice can be immunized with DNA encoding the antigen or fragments thereof. In the event that immunizations using a purified or enriched preparation of the antigen do not result in antibodies, mice can also be immunized with cells expressing the antigen, e.g., a cell line, to promote immune responses.

The immune response can be monitored over the course of the immunization protocol with plasma and serum samples being obtained by tail vein or retroorbital bleeds. Mice with sufficient titers of immunoglobulin can be used for fusions. Mice can be boosted intraperitonealy or intravenously with antigen expressing cells 3 days before sacrifice and removal of the spleen to increase the rate of specific antibody secreting hybridomas.

To generate hybridomas producing monoclonal antibodies, splenocytes and lymph node cells from immunized mice can be isolated and fused to an appropriate immortalized cell line, such as a mouse myeloma cell line. The resulting hybridomas can then be screened for the production of antigen-specific antibodies. Individual wells can then be screened by ELISA for antibody secreting hybridomas. By Immunofluorescence and FACS analysis using antigen expressing cells, antibodies with specificity for the antigen can be identified. The antibody secreting hybridomas can be replated, screened again, and if still positive for monoclonal antibodies can be subcloned by limiting dilution. The stable subclones can then be cultured in vitro to generate antibody in tissue culture medium for characterization.

Antibodies also can be produced in a host cell transfectoma using, for example, a combination of recombinant DNA techniques and gene transfection methods as are well known in the art (Morrison, S. (1985) Science 229: 1202).

For example, in one embodiment, the gene(s) of interest, e.g., antibody genes, can be ligated into an expression vector such as a eukaryotic expression plasmid such as used by the GS gene expression system disclosed in WO 87/04462, WO 89/01036 and EP 338 841 or other expression systems well known in the art. The purified plasmid with the cloned antibody genes can be introduced in eukaryotic host cells such as CHO cells, NS/0 cells, HEK293T cells or HEK293 cells or alternatively other eukaryotic cells like plant derived cells, fungal or yeast cells. The method used to introduce these genes can be methods described in the art such as electroporation, lipofectine, lipofectamine or others. After introduction of these antibody genes in the host cells, cells expressing the antibody can be identified and selected. These cells represent the transfectomas which can then be amplified for their expression level and upscaled to produce antibodies. Recombinant antibodies can be isolated and purified from these culture supernatants and/or cells.

Alternatively, the cloned antibody genes can be expressed in other expression systems, including prokaryotic cells, such as microorganisms, e.g. E. coli. Furthermore, the antibodies can be produced in transgenic non-human animals, such as in milk from sheep and rabbits or in eggs from hens, or in transgenic plants; see e.g. Verma, R., et al. (1998) J. Immunol. Meth. 216: 165-181; Pollock, et al. (1999) J. Immunol. Meth. 231: 147-157; and Fischer, R., et al. (1999) Biol. Chem. 380: 825-839.

### Chimerization

Murine antibodies are highly immunogenic in man when repetitively applied leading to reduction of the therapeutic effect. The main immunogenicity is mediated by the heavy chain constant regions. The immunogenicity of murine antibodies in man can be reduced or completely avoided if respective antibodies are chimerized or humanized. Chimeric antibodies are antibodies, the different portions of which are derived from different animal species, such as those having a variable region derived from a murine antibody and a human immunoglobulin constant region. Chimerisation of antibodies is achieved by joining of the variable regions of the murine antibody heavy and light chain with the constant region of human heavy and light chain (e.g. as described by Kraus et al., in Methods in Molecular Biology series, Recombinant antibodies for cancer therapy ISBN-0-89603-918-8). In a preferred embodiment chimeric antibodies are generated by joining human kappa-light chain constant region to murine light chain variable region. In an also preferred embodiment chimeric antibodies can be generated by joining human lambda-light chain constant region to murine light chain variable region. The preferred heavy chain constant regions for generation of chimeric antibodies are IgG1, IgG3 and IgG4. Other preferred heavy chain constant regions for generation of chimeric antibodies are IgG2, IgA, IgD and IgM.

### Humanization

Antibodies interact with target antigens predominantly through amino acid residues that are located in the six heavy and light chain complementarity determining regions (CDRs). For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann, L. et al. (1998) Nature 332: 323-327; Jones, P. et al. (1986) Nature 321: 522-525; and Queen, C. et al. (1989) Proc. Natl. Acad. Sci. U. S. A. 86: 10029-10033). Such framework sequences can be obtained from public DNA databases that include germline antibody gene sequences. These germline sequences will differ from mature antibody gene sequences because they will not include completely assembled variable genes, which are formed by V (D) J joining during B cell maturation. Germline gene sequences will also differ from the sequences of a high affinity secondary repertoire antibody at individual evenly across the variable region.

The ability of antibodies to bind an antigen can be determined using standard binding assays (e.g., ELISA, Western Blot, Immunofluorescence and flow cytometric analysis).

To purify antibodies, selected hybridomas can be grown in two-liter spinner-flasks for monoclonal antibody purification. Alternatively, antibodies can be produced in dialysis based bioreactors. Supernatants can be filtered and, if necessary, concentrated before affinity chromatography with protein G-sepharose or protein A-sepharose. Eluted IgG can be checked by gel electrophoresis and high performance liquid chromatography to ensure purity. The buffer solution can be exchanged into PBS, and the concentration can be determined by OD280 using 1.43 extinction coefficient. The monoclonal antibodies can be aliquoted and stored at -80°C.

To determine if the selected monoclonal antibodies bind to unique epitopes, site-directed or multi-site directed mutagenesis can be used.

To determine the isotype of antibodies, isotype ELISAs with various commercial kits (e.g. Zymed, Roche Diagnostics) can be performed. Wells of microtiter plates can be coated with anti-mouse Ig. After blocking, the plates are reacted with monoclonal antibodies or purified isotype controls, at ambient temperature for two hours. The wells can then be reacted with either mouse IgG1, IgG2a, IgG2b or IgG3, IgA or mouse IgM-specific peroxidase-conjugated probes. After washing, the plates can be developed with ABTS substrate (1 mg/ml) and analyzed at OD of 405-650. Alternatively, the IsoStrip Mouse Monoclonal Antibody Isotyping Kit (Roche, Cat. No. 1493027) may be used as described by the manufacturer.

In order to demonstrate presence of antibodies in sera of immunized mice or binding of monoclonal antibodies to living cells expressing antigen, flow cytometry can be used. Cell lines expressing naturally or after transfection antigen and negative controls lacking antigen expression (grown under standard growth conditions) can be mixed with various concentrations of monoclonal antibodies in hybridoma supernatants or in PBS containing 1% FBS, and can be incubated at 4°C for 30 min. After washing, the APC- or Alexa647-labeled anti IgG antibody can bind to antigen-bound monoclonal antibody under the same conditions as the primary antibody staining. The samples can be analyzed by flow cytometry with a FACS instrument using light and side scatter properties to gate on single, living cells. In order to distinguish antigen-specific monoclonal antibodies from non-specific binders in a single measurement, the method of co-transfection can be employed. Cells transiently transfected with plasmids encoding antigen and a fluorescent marker can be stained as described above. Transfected cells can be detected in a different fluorescence channel than antibody-stained cells. As the majority of transfected cells express both transgenes, antigen-specific monoclonal antibodies bind preferentially to fluorescence marker expressing cells, whereas non-specific antibodies bind in a comparable ratio to non-transfected cells. An alternative assay using fluorescence microscopy may be used in addition to or instead of the flow cytometry assay. Cells can be stained exactly as described above and examined by fluorescence microscopy.

In order to demonstrate presence of antibodies in sera of immunized mice or binding of monoclonal antibodies to living cells expressing antigen, immunofluorescence microscopy analysis can be used. For example, cell lines expressing either spontaneously or after transfection antigen and negative controls lacking antigen expression are grown in chamber slides under standard growth conditions in DMEM/F12 medium, supplemented with 10 % fetal calf serum (FCS), 2 mM L-glutamine, 100 IU/ml penicillin and 100 µg/ml streptomycin. Cells can then be fixed with methanol or paraformaldehyde or left untreated. Cells can then be reacted with monoclonal antibodies against the antigen for 30 min. at 25°C. After washing, cells can be reacted with an Alexa555-labelled anti-mouse IgG secondary antibody (Molecular Probes) under the same conditions. Cells can then be examined by fluorescence microscopy.

Cell extracts from cells expressing antigen and appropriate negative controls can be prepared and subjected to sodium dodecyl sulfate (SDS) polyacrylamide gel electrophoresis. After electrophoresis, the separated antigens will be transferred to nitrocellulose membranes, blocked, and probed with the monoclonal antibodies to be tested. IgG binding can be detected using anti-mouse IgG peroxidase and developed with ECL substrate.

Antibodies can be further tested for reactivity with antigen by Immunohistochemistry in a manner well known to the skilled person, e.g. using paraformaldehyde or acetone fixed cryosections or paraffin embedded tissue sections fixed with paraformaldehyde from non-cancer tissue or cancer tissue samples obtained from patients during routine surgical procedures or from mice carrying xenografted tumors inoculated with cell lines expressing spontaneously or after transfection antigen. For immunostaining, antibodies reactive to antigen can be incubated followed by horseradish-peroxidase conjugated goat anti-mouse or goat anti-rabbit antibodies (DAKO) according to the vendors' instructions.

Antibodies can be tested for their ability to mediate phagocytosis and killing of cells expressing a tumor antigen. The testing of monoclonal antibody activity in vitro will provide an initial screening prior to testing in vivo models.

### Antibody dependent cell-mediated cytotoxicity (ADCC)

Briefly, polymorphonuclear cells (PMNs), NK cells, monocytes, mononuclear cells or other effector cells, from healthy donors can be purified by Ficoll Hypaque density centrifugation, followed by lysis of contaminating erythrocytes. Washed effector cells can be suspended in RPMI supplemented with 10% heat-inactivated fetal calf serum or, alternatively with 5% heat-inactivated human serum and mixed with ⁵¹Cr labeled target cells expressing a tumor antigen, at various ratios of effector cells to target cells. Alternatively, the target cells may be labeled with a fluorescence enhancing ligand (BATDA). A highly fluorescent chelate of Europium with the enhancing ligand which is released from dead cells can be measured by a fluorometer. Another alternative technique may utilize the transfection of target cells with luciferase. Added lucifer yellow may then be oxidated by viable cells only. Purified anti- tumor antigen IgGs can then be added at various concentrations. Irrelevant human IgG can be used as negative control. Assays can be carried out for 4 to 20 hours at 37°C depending on the effector cell type used. Samples can be assayed for cytolysis by measuring ⁵¹Cr release or the presence of the EuTDA chelate in the culture supernatant. Alternatively, luminescence resulting from the oxidation of lucifer yellow can be a measure of viable cells.

Anti-tumor antigen monoclonal antibodies can also be tested in various combinations to determine whether cytolysis is enhanced with multiple monoclonal antibodies.

### Complement dependent cytotoxicity (CDC)

Monoclonal anti-tumor antigen antibodies can be tested for their ability to mediate CDC using a variety of known techniques. For example, serum for complement can be obtained from blood in a manner known to the skilled person. To determine the CDC activity of mAbs, different methods can be used. ⁵¹Cr release can for example be measured or elevated membrane permeability can be assessed using a propidium iodide (PI) exclusion assay. Briefly, target cells can be washed and 5 x 10⁵/ml can be incubated with various concentrations of mAb for 10-30 min. at room temperature or at 37°C. Serum or plasma can then be added to a final concentration of 20% (v/v) and the cells incubated at 37°C for 20-30 min. All cells from each sample can be added to the PI solution in a FACS tube. The mixture can then be analyzed immediately by flow cytometry analysis using FACSArray.

In an alternative assay, induction of CDC can be determined on adherent cells. In one embodiment of this assay, cells are seeded 24 h before the assay with a density of 3 x 10⁴/well in tissue-culture flat-bottom microtiter plates. The next day growth medium is removed and the cells are incubated in triplicates with antibodies. Control cells are incubated with growth medium or growth medium containing 0.2% saponin for the determination of background lysis and maximal lysis, respectively. After incubation for 20 min. at room temperature supernatant is removed and 20% (v/v) human plasma or serum in DMEM (prewarmed to 37°C) is added to the cells and incubated for another 20 min. at 37°C. All cells from each sample are added to propidium iodide solution (10 µg/ml). Then, supernatants are replaced by PBS containing 2.5 µg/ml ethidium bromide and fluorescence emission upon excitation at 520 nm is measured at 600 nm using a Tecan Safire. The percentage specific lysis is calculated as follows: % specific lysis = (fluorescence sample-fluorescence background)/ (fluorescence maximal lysis-fluorescence background) x 100.

### Induction of apoptosis and inhibition of cell proliferation by monoclonal antibodies

To test for the ability to initiate apoptosis, monoclonal anti-tumor antigen antibodies can, for example, be incubated with tumor antigen positive tumor cells or tumor antigen transfected tumor cells at 37°C for about 20 hours. The cells can be harvested, washed in Annexin-V binding buffer (BD biosciences), and incubated with Annexin V conjugated with FITC or APC (BD biosciences) for 15 min. in the dark. All cells from each sample can be added to PI solution (10 µg/ml in PBS) in a FACS tube and assessed immediately by flow cytometry (as above). Alternatively, a general inhibition of cell-proliferation by monoclonal antibodies can be detected with commercially available kits. The DELFIA Cell Proliferation Kit (Perkin-Elmer, Cat. No. AD0200) is a non-isotopic immunoassay based on the measurement of 5-bromo-2'-deoxyuridine (BrdU) incorporation during DNA synthesis of proliferating cells in microplates. Incorporated BrdU is detected using europium labelled monoclonal antibody. To allow antibody detection, cells are fixed and DNA denatured using Fix solution. Unbound antibody is washed away and DELFIA inducer is added to dissociate europium ions from the labelled antibody into solution, where they form highly fluorescent chelates with components of the DELFIA Inducer. The fluorescence measured - utilizing time-resolved fluorometry in the detection - is proportional to the DNA synthesis in the cell of each well.

### Preclinical studies

Binding agents described herein also can be tested in an *in vivo* model (e.g. in immune deficient mice carrying xenografted tumors inoculated with cell lines expressing a tumor antigen to determine their efficacy in controlling growth of tumor antigen-expressing tumor cells.

In vivo studies after xenografting tumor antigen expressing tumor cells into immunocompromised mice or other animals can be performed using antibodies described herein. Antibodies can be administered to tumor free mice followed by injection of tumor cells to measure the effects of the antibodies to prevent formation of tumors or tumor-related symptoms. Antibodies can be administered to tumor-bearing mice to determine the therapeutic efficacy of respective antibodies to reduce tumor growth, metastasis or tumor related symptoms. Antibody application can be combined with application of other substances as cystostatic drugs, growth factor inhibitors, cell cycle blockers, angiogenesis inhibitors or other antibodies to determine synergistic efficacy and potential toxicity of combinations. To analyze toxic side effects mediated by antibodies animals can be inoculated with antibodies or control reagents and thoroughly investigated for symptoms possibly related to tumor antigen-antibody therapy. Possible side effects of in vivo application of tumor antigen antibodies particularly include toxicity at tumor antigen expressing tissues. Antibodies recognizing a tumor antigen in human and in other species, e.g. mice, are particularly useful to predict potential side effects mediated by application of monoclonal tumor antigen-antibodies in humans.

Mapping of epitopes recognized by antibodies can be performed as described in detail in "Epitope Mapping Protocols (Methods in Molecular Biology) by Glenn E. Morris ISBN-089603-375-9 and in "Epitope Mapping: A Practical Approach" Practical Approach Series, 248 by Olwyn M. R. Westwood, Frank C. Hay.

The compounds and agents described herein may be administered in the form of any suitable pharmaceutical composition.

Pharmaceutical compositions are preferably sterile and contain an effective amount of the antibodies described herein and optionally of further agents as discussed herein to generate the desired reaction or the desired effect.

Pharmaceutical compositions are usually provided in a uniform dosage form and may be prepared in a manner known per se. A pharmaceutical composition may e.g. be in the form of a solution or suspension.

A pharmaceutical composition may comprise salts, buffer substances, preservatives, carriers, diluents and/or excipients all of which are preferably pharmaceutically acceptable. The term "pharmaceutically acceptable" refers to the non-toxicity of a material which does not interact with the action of the active component of the pharmaceutical composition.

Salts which are not pharmaceutically acceptable may be used for preparing pharmaceutically acceptable salts and are included in the invention. Pharmaceutically acceptable salts of this kind comprise in a non limiting way those prepared from the following acids: hydrochloric, hydrobromic, sulfuric, nitric, phosphoric, maleic, acetic, salicylic, citric, formic, malonic, succinic acids, and the like. Pharmaceutically acceptable salts may also be prepared as alkali metal salts or alkaline earth metal salts, such as sodium salts, potassium salts or calcium salts.

Suitable buffer substances for use in a pharmaceutical composition include acetic acid in a salt, citric acid in a salt, boric acid in a salt and phosphoric acid in a salt.

Suitable preservatives for use in a pharmaceutical composition include benzalkonium chloride, chlorobutanol, paraben and thimerosal.

An injectible formulation may comprise a pharmaceutically acceptable excipient such as Ringer lactate.

The term "carrier" refers to an organic or inorganic component, of a natural or synthetic nature, in which the active component is combined in order to facilitate, enhance or enable application. According to the invention, the term "carrier" also includes one or more compatible solid or liquid fillers, diluents or encapsulating substances, which are suitable for administration to a patient.

Possible carrier substances for parenteral administration are e.g. sterile water, Ringer, Ringer lactate, sterile sodium chloride solution, polyalkylene glycols, hydrogenated naphthalenes and, in particular, biocompatible lactide polymers, lactide/glycolide copolymers or polyoxyethylene/polyoxy- propylene copolymers.

The term "excipient" when used herein is intended to indicate all substances which may be present in a pharmaceutical composition and which are not active ingredients such as, e.g., carriers, binders, lubricants, thickeners, surface active agents, preservatives, emulsifiers, buffers, flavoring agents, or colorants.

The agents and compositions described herein may be administered via any conventional route, such as by parenteral administration including by injection or infusion. Administration is preferably parenterally, e.g. intravenously, intraarterially, subcutaneously, intradermally or intramuscularly.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The agents and compositions described herein are administered in effective amounts. An "effective amount" refers to the amount which achieves a desired reaction or a desired effect alone or together with further doses. In the case of treatment of a particular disease or of a particular condition, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition. In particular, the term "effective amount" refers to the amount of a therapy that is sufficient to result in the prevention of the development, recurrence, or onset of cancer and one or more symptoms thereof, reduce the severity, the duration of cancer, ameliorate one or more symptoms of cancer, prevent the advancement of cancer, cause regression of cancer, and/or prevent cancer metastases. In an embodiment of the invention, the amount of a therapy is effective to achieve a stabilization, reduction or elimination of the cancer stem cell population and/or eradication, removal, or control of primary cancer, metastatic cancer and/or recurrent cancer.

An effective amount of an agent or composition described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the agents described herein may depend on various of such parameters. In the case that a reaction in a patient is insufficient with an initial dose, higher doses (or effectively higher doses achieved by a different, more localized route of administration) may be used.

The agents and compositions provided herein may be used alone or in combination with conventional therapeutic regimens such as surgery, irradiation, chemotherapy and/or bone marrow transplantation (autologous, syngeneic, allogeneic or unrelated).

Treatment of cancer represents a field where combination strategies are especially desirable since frequently the combined action of two, three, four or even more cancer drugs/therapies generates synergistic effects which are considerably stronger than the impact of a monotherapeutic approach. Thus, in another embodiment of the present invention, a cancer treatment may be effectively combined with various other drugs. Among those are e.g. combinations with conventional tumor therapies, multi-epitope strategies, additional immunotherapy, and treatment approaches targeting angiogenesis or apoptosis (for review see e.g. Andersen et al. 2008: Cancer treatment: the combination of vaccination with other therapies. Cancer Immunology Immunotherapy, 57(11): 1735-1743.) Sequential administration of different agents may inhibit cancer cell growth at different check points, while other agents may e.g. inhibit neo-angiogenesis, survival of malignant cells or metastases, potentially converting cancer into a chronic disease. The following list provides some non-limiting examples of anti-cancer drugs and therapies which can be used in combination with the present invention:

### 1. Chemotherapy

Chemotherapy is the standard of care for multiple types of cancer. The most common chemotherapy agents act by killing cells that divide rapidly, one of the main properties of cancer cells. Thus, a combination with conventional chemotherapeutic drugs such as e.g. alkylating agents, antimetabolites, anthracyclines, plant alkaloids, topoisomerase inhibitors, and other antitumour agents which either affect cell division or DNA synthesis may significantly improve the therapeutic effects of the present invention by clearing suppressor cells, reboot of the immune system, by rendering tumor cells more susceptible to immune mediated killing, or by additional activation of cells of the immune system. A synergistic anti-cancer action of chemotherapeutic and vaccination-based immunotherapeutic drugs has been demonstrated in multiple studies (see e.g. Quoix et al. 2011: Therapeutic vaccination with TG4010 and first-line chemotherapy in advanced non-small-cell lung cancer: a controlled phase 2B trial. Lancet Oncol. 12(12): 1125-33.; see also Liseth et al. 2010: Combination of intensive chemotherapy and anticancer vaccines in the treatment of human malignancies: the hematological experience. J Biomed Biotechnol. 2010: 6920979; see also Hirooka et al 2009: A combination therapy of gemcitabine with immunotherapy for patients with inoperable locally advanced pancreatic cancer. Pancreas 38(3): e69-74). There are hundreds of chemotherapeutic drugs available which are basically suitable for combination therapies. Some (non-limiting) examples of chemotherapeutic drugs which can be combined with the present invention are carboplatin (Paraplatin), cisplatin (Platinol, Platinol-AQ), cyclophosphamide (Cytoxan, Neosar), docetaxel (Taxotere), doxorubicin (Adriamycin), erlotinib (Tarceva), etoposide (VePesid), fluorouracil (5-FU), gemcitabine (Gemzar), imatinib mesylate (Gleevec), irinotecan (Camptosar), methotrexate (Folex, Mexate, Amethopterin), paclitaxel (Taxol, Abraxane), sorafinib (Nexavar), sunitinib (Sutent), topotecan (Hycamtin), vincristine (Oncovin, Vincasar PFS), and vinblastine (Velban).

### 2. Surgery

Cancer surgery - an operation to remove the tumor - remains the foundation of cancer treatment. Surgery can be combined with other cancer treatments in order to delete any remaining tumor cells. Combining surgical methods with subsequent immunotherapeutic treatment is a promising approach which has been demonstrated countless times.

### 3. Radiation

Radiation therapy remains an important component of cancer treatment with approximately 50% of all cancer patients receiving radiation therapy during their course of illness. The main goal of radiation therapy is to deprive cancer cells of their multiplication (cell division) potential. The types of radiation used to treat cancer are photons radiation (x-rays and gamma rays) and particle radiations (electron, proton and neutron beams.) There are two ways to deliver the radiation to the location of the cancer. External beam radiation is delivered from outside the body by aiming high-energy rays (photons, protons or particle radiation) to the location of the tumor. Internal radiation or brachytherapy is delivered from inside the body by radioactive sources, sealed in catheters or seeds directly into the tumor site. Radiation therapy techniques which are applicable in combination with the present invention are e.g. fractionation (radiation therapy delivered in a fractionated regime, e.g. daily fractions of 1.5 to 3 Gy given over several weeks), 3D conformal radiotherapy (3DCRT; delivering radiation to the gross tumor volume), intensity modulated radiation therapy (IMRT; computer-controlled intensity modulation of multiple radiation beams), image guided radiotherapy (IGRT; a technique comprising pre-radiotherapy imaging which allows for correction), and stereotactic body radiation therapy (SRBT, delivers very high individual doses of radiation over only a few treatment fractions). For a radiation therapy review see Baskar et al. 2012: Cancer and radiation therapy: current advances and future directions. Int. J Med Sci. 9(3): 193-199.

### 4. Antibodies

Antibodies (preferably monoclonal antibodies) achieve their therapeutic effect against cancer cells through various mechanisms. They can have direct effects in producing apoptosis or programmed cell death. They can block components of signal transduction pathways such as e.g. growth factor receptors, effectively arresting proliferation of tumor cells. In cells that express monoclonal antibodies, they can bring about anti-idiotype antibody formation. Indirect effects include recruiting cells that have cytotoxicity, such as monocytes and macrophages. This type of antibody-mediated cell kill is called antibody-dependent cell mediated cytotoxicity (ADCC). Antibodies also bind complement, leading to direct cell toxicity, known as complement dependent cytotoxicity (CDC). Combining surgical methods with immunotherapeutic drugs or methods is an successful approach, as e.g. demonstrated in Gadri et al. 2009: Synergistic effect of dendritic cell vaccination and anti-CD20 antibody treatment in the therapy of murine lymphoma. J Immunother. 32(4): 333-40. The following list provides some non-limiting examples of anti-cancer antibodies and potential antibody targets (in brackets) which can be used in combination with the present invention: Abagovomab (CA-125), Abciximab (CD41), Adecatumumab (EpCAM), Afutuzumab (CD20), Alacizumab pegol (VEGFR2), Altumomab pentetate (CEA), Amatuximab (MORAb-009), Anatumomab mafenatox (TAG-72), Apolizumab (HLA-DR), Arcitumomab (CEA), Bavituximab (phosphatidylserine), Bectumomab (CD22), Belimumab (BAFF), Bevacizumab (VEGF-A), Bivatuzumab mertansine (CD44 v6), Blinatumomab (CD19), Brentuximab vedotin (CD30 TNFRSF8), Cantuzumab mertansin (mucin CanAg), Cantuzumab ravtansine (MUC1), Capromab pendetide (prostatic carcinoma cells), Carlumab (CNT0888), Catumaxomab (EpCAM, CD3), Cetuximab (EGFR), Citatuzumab bogatox (EpCAM), Cixutumumab (IGF-1 receptor), Claudiximab (Claudin), Clivatuzumab tetraxetan (MUC1), Conatumumab (TRAIL-R2), Dacetuzumab (CD40), Dalotuzumab (insulin-like growth factor I receptor), Denosumab (RANKL), Detumomab (B-lymphoma cell), Drozitumab (DR5), Ecromeximab (GD3 ganglioside), Edrecolomab (EpCAM), Elotuzumab (SLAMF7), Enavatuzumab (PDL192), Ensituximab (NPC-1C), Epratuzumab (CD22), Ertumaxomab (HER2/neu, CD3), Etaracizumab (integrin αvβ3), Farletuzumab (folate receptor 1), FBTA05 (CD20), Ficlatuzumab (SCH 900105), Figitumumab (IGF-1 receptor), Flanvotumab (glycoprotein 75), Fresolimumab (TGF-β), Galiximab (CD80), Ganitumab (IGF-I), Gemtuzumab ozogamicin (CD33), Gevokizumab (IL-1β), Girentuximab (carbonic anhydrase 9 (CA-IX)), Glembatumumab vedotin (GPNMB), Ibritumomab tiuxetan (CD20), Icrucumab (VEGFR-1), Igovoma (CA-125), Indatuximab ravtansine (SDC1), Intetumumab (CD51), Inotuzumab ozogamicin (CD22), Ipilimumab (CD152), Iratumumab (CD30), Labetuzumab (CEA), Lexatumumab (TRAIL-R2), Libivirumab (hepatitis B surface antigen), Lintuzumab (CD33), Lorvotuzumab mertansine (CD56), Lucatumumab (CD40), Lumiliximab (CD23), Mapatumumab (TRAIL-R1), Matuzumab (EGFR), Mepolizumab (IL-5), Milatuzumab (CD74), Mitumomab (GD3 ganglioside), Mogamulizumab (CCR4), Moxetumomab pasudotox (CD22), Nacolomab tafenatox (C242 antigen), Naptumomab estafenatox (5T4), Narnatumab (RON), Necitumumab (EGFR), Nimotuzumab (EGFR), Nivolumab (IgG4), Ofatumumab (CD20), Olaratumab (PDGF-R α), Onartuzumab (human scatter factor receptor kinase), Oportuzumab monatox (EpCAM), Oregovomab (CA-125), Oxelumab (OX-40), Panitumumab (EGFR), Patritumab (HER3), Pemtumoma (MUC1), Pertuzumab (HER2/neu), Pintumomab (adenocarcinoma antigen), Pritumumab (vimentin), Racotumomab (N-glycolylneuraminic acid), Radretumab (fibronectin extra domain-B), Rafivirumab (rabies virus glycoprotein), Ramucirumab (VEGFR2), Rilotumumab (HGF), Rituximab (CD20), Robatumumab (IGF-1 receptor), Samalizumab (CD200), Sibrotuzumab (FAP), Siltuximab (IL-6), Tabalumab (BAFF), Tacatuzumab tetraxetan (alpha-fetoprotein), Taplitumomab paptox (CD19), Tenatumomab (tenascin C), Teprotumumab (CD221), Ticilimumab (CTLA-4), Tigatuzumab (TRAIL-R2), TNX-650 (IL-13), Tositumomab (CD20), Trastuzumab (HER2/neu), TRBS07 (GD2), Tremelimumab (CTLA-4), Tucotuzumab celmoleukin (EpCAM), Ublituximab (MS4A1), Urelumab (4-1BB), Volociximab (integrin α5β1), Votumumab (tumor antigen CTAA16.88), Zalutumumab (EGFR), Zanolimumab (CD4).

### 5. Cytokines, chemokines, costimulatory molecules, fusion proteins

Combined usage of the antigen-coding pharmaceutical compositions of the present invention with cytokines, chemokines, costimulatory molecules and/or fusion proteins thereof to evoke beneficial immune modulation or tumor inhibition effects is another embodiment of the present invention. In order to increase the infiltration of immune cells into the tumor and facilitate the movement of antigen-presenting cells to tumor-draining lymph nodes, various chemokines with C, CC, CXC and CX3C structures might be used. Some of the most promising chemokines are e.g CCR7 and its ligands CCL19 and CCL21, furthermore CCL2, CCL3, CCL5, and CCL16. Other examples are CXCR4, CXCR7 and CXCL12. Furthermore, costimulatory or regulatory molecules such as e.g. B7 ligands (B7.1 and B7.2) are useful. Also useful are other cytokines such as e.g. interleukins especially (e.g. IL-1 to IL17), interferons (e.g. IFNalpha1 to IFNalpha8, IFNalpha10, IFNalpha13, IFNalpha14, IFNalpha16, IFNalpha17, IFNalpha21, IFNbeta1, IFNW, IFNE1 and IFNK), hematopoietic factors, TGFs (e.g. TGF-α, TGF-β, and other members of the TGF family), finally members of the tumor necrosis factor family of receptors and their ligands as well as other stimulatory molecules, comprising but not limited to 4-1BB, 4-1BB-L, CD137, CD137L, CTLA-4GITR, GITRL, Fas, Fas-L, TNFR1, TRAIL-R1, TRAIL-R2, p75NGF-R, DR6, LT.beta.R, RANK, EDAR1, XEDAR, Fn114, Troy/Trade, TAJ, TNFRII, HVEM, CD27, CD30, CD40, 4-1 BB, OX40, GITR, GITRL, TACI, BAFF-R, BCMA, RELT, and CD95 (Fas/APO-1), glucocorticoid-induced TNFR-related protein, TNF receptor-related apoptosis-mediating protein (TRAMP) and death receptor-6 (DR6). Especially CD40/CD40L and OX40/OX40L are important targets for combined immunotherapy because of their direct impact on T cell survival and proliferation. For a review see Lechner et al. 2011: Chemokines, costimulatory molecules and fusion proteins for the immunotherapy of solid tumors. Immunotherapy 3 (11), 1317-1340.

### 6. Bacterial treatments

Researchers have been using anaerobic bacteria, such as Clostridium novyi, to consume the interior of oxygen-poor tumours. These should then die when they come in contact with the tumour's oxygenated sides, meaning they would be harmless to the rest of the body. Another strategy is to use anaerobic bacteria that have been transformed with an enzyme that can convert a non-toxic prodrug into a toxic drug. With the proliferation of the bacteria in the necrotic and hypoxic areas of the tumour, the enzyme is expressed solely in the tumour. Thus, a systemically applied prodrug is metabolised to the toxic drug only in the tumour. This has been demonstrated to be effective with the nonpathogenic anaerobe Clostridium sporogenes.

### 7. Kinase inhibitors

Another large group of potential targets for complementary cancer therapy comprises kinase inhibitors, because the growth and survival of cancer cells is closely interlocked with the deregulation of kinase activity. To restore normal kinase activity and therefor reduce tumor growth a broad range of inhibitors is in used. The group of targeted kinases comprises receptor tyrosine kinases e.g. BCR-ABL, B-Raf, EGFR, HER-2/ErbB2, IGF-IR, PDGFR-α, PDGFR-β, c-Kit, Flt-4, Flt3, FGFR1, FGFR3, FGFR4, CSF1R, c-Met, RON, c-Ret, ALK, cytoplasmic tyrosine kinases e.g. c-SRC, c-YES, Abl, JAK-2, serine/threonine kinases e.g. ATM, Aurora A & B, CDKs, mTOR, PKCi, PLKs, b-Raf, S6K, STK11/LKB1 and lipid kinases e.g. PI3K, SK1. Small molecule kinase inhibitors are e.g. PHA-739358, Nilotinib, Dasatinib, and PD166326, NSC 743411, Lapatinib (GW-572016), Canertinib (CI-1033), Semaxinib (SU5416), Vatalanib (PTK787/ZK222584), Sutent (SU11248), Sorafenib (BAY 43-9006) and Leflunomide (SU101). For more information see e.g. Zhang et al. 2009: Targeting cancer with small molecule kinase inhibitors. Nature Reviews Cancer 9, 28-39.

### 8. Toll-like receptors

The members of the Toll-like receptor (TLRs) family are an important link between innate and adaptive immunity and the effect of many adjuvants rely on the activation of TLRs. A large number of established vaccines against cancer incorporate ligands for TLRs for boosting vaccine responses. Besides TLR2, TLR3, TLR4 especially TLR7 and TLR 8 have been examined for cancer therapy in passive immunotherapy approaches. The closely related TLR7 and TLR8 contribute to antitumor responses by affecting immune cells, tumor cells, and the tumor microenvironment and may be activated by nucleoside analogue structures. All TLR's have been used as stand-alone immunotherapeutics or cancer vaccine adjuvants and may be synergistically combined with the formulations and methods of the present invention. For more information see van Duin et al. 2005: Triggering TLR signaling in vaccination. Trends in Immunology, 27(1):49-55.

### 9. Angiogenesis inhibitors

In addition to therapies which target immune modulatory receptors affected by tumor-mediated escape mechanisms and immune suppression there are therapies which target the tumor environment. Angiogenesis inhibitors prevent the extensive growth of blood vessels (angiogenesis) that tumors require to survive. The angiogenesis promoted by tumor cells to meet their increasing nutrient and oxygen demands for example can be blocked by targeting different molecules. Non-limiting examples of angiogenesis-mediating molecules or angiogenesis inhibitors which may be combined with the present invention are soluble VEGF (VEGF isoforms VEGF121 and VEGF165, receptors VEGFR1, VEGFR2 and co-receptors Neuropilin-1 and Neuropilin-2) 1 and NRP-1, angiopoietin 2, TSP-1 and TSP-2, angiostatin and related molecules, endostatin, vasostatin, calreticulin, platelet factor-4, TIMP and CDAI, Meth-1 and Meth-2, IFN-α, -β and -y, CXCL10, IL-4, -12 and -18, prothrombin (kringle domain-2), antithrombin III fragment, prolactin, VEGI, SPARC, osteopontin, maspin, canstatin, proliferin-related protein, restin and drugs like e.g. bevacizumab, itraconazole, carboxyamidotriazole, TNP-470, CM101, IFN-α,, platelet factor-4, suramin, SU5416, thrombospondin, VEGFR antagonists, angiostatic steroids + heparin, cartilage-derived angiogenesis Inhibitory factor, matrix metalloproteinase inhibitors, 2-methoxyestradiol, tecogalan, tetrathiomolybdate, thalidomide, thrombospondin, prolactina Vβ3 inhibitors, linomide, tasquinimod, For review see Schoenfeld and Dranoff 2011: Anti-angiogenesis immunotherapy. Hum Vaccin. (9):976-81.

### 10. Small molecule targeted therapy drugs

Small molecule targeted therapy drugs are generally inhibitors of enzymatic domains on mutated, overexpressed, or otherwise critical proteins within the cancer cell. Prominent and non-limiting examples are the tyrosine kinase inhibitors imatinib (Gleevec/Glivec) and gefitinib (Iressa). The use of small molecules e.g. sunitinib malate and/or sorafenib tosylate targeting some kinases in combination with vaccines for cancer therapy is also described in previous patent application US2009004213.

### 11. Virus-based vaccines

There are a number of virus-based cancer vaccines available or under development which can be used in a combined therapeutic approach together with the formulations of the present invention. One advantage of the use of such viral vectors is their intrinsic ability to initiate immune responses, with inflammatory reactions occurring as a result of the viral infection creating the danger signal necessary for immune activation. An ideal viral vector should be safe and should not introduce an anti-vector immune response to allow for boosting antitumour specific responses. Recombinant viruses such as vaccinia viruses, herpes simplex viruses, adenoviruses, adeno-associated viruses, retroviruses and avipox viruses have been used in animal tumour models and based on their encouraging results, human clinical trials have been initiated. Especially important virus-based vaccines are virus-like particles (VLPs), small particles that contain certain proteins from the outer coat of a virus. Virus-like particles do not contain any genetic material from the virus and cannot cause an infection but they can be constructed to present tumor antigens on their coat. VLPs can be derived from various viruses such as e.g. the hepatitis B virus or other virus families including Parvoviridae (e.g. adeno-associated virus), Retroviridae (e.g. HIV), and Flaviviridae (e.g. Hepatitis C virus). For a general review see Sorensen and Thompsen 2007: Virus-based immunotherapy of cancer: what do we know and where are we going? APMIS 115(11):1177-93; virus-like particles against cancer are reviewed in Buonaguro et al. 2011: Developments in virus-like particle-based vaccines for infectious diseases and cancer. Expert Rev Vaccines 10(11):1569-83; and in Guillén et al. 2010: Virus-like particles as vaccine antigens and adjuvants: application to chronic disease, cancer immunotherapy and infectious disease preventive strategies. Procedia in Vaccinology 2 (2), 128-133.

### 12. Multi-epitope strategies

The use of multi epitopes shows promising results for vaccination. Fast sequencing technologies combined with intelligent algorithms systems allow the exploitation of the tumor mutanome and may provide multi epitopes for individualized vaccines which can be combined with the present invention. For more information see 2007: Vaccination of metastatic colorectal cancer patients with matured dendritic cells loaded with multiple major histocompatibility complex class I peptides. J Immunother 30: 762-772; furthermore Castle et al. 2012: Exploiting the mutanome for tumor vaccination. Cancer Res 72 (5):1081-91.

### 13. Adoptive T cell transfer

For example, a combination of a tumor antigen vaccination and T cell transfer is described in: Rapoport et al. 2011: Combination immunotherapy using adoptive T-cell transfer and tumor antigen vaccination on the basis of hTERT and survivin after ASCT for myeloma. Blood 117(3):788-97.

### 14. Peptide-based target therapies

Peptides can bind to cell surface receptors or affected extracellular matrix surrounding the tumor. Radionuclides which are attached to these peptides (e.g. RGDs) eventually kill the cancer cell if the nuclide decays in the vicinity of the cell. Especially oligo- or multimers of these binding motifs are of great interest, since this can lead to enhanced tumor specificity and avidity. For non-limiting examples see Yamada 2011: Peptide-based cancer vaccine therapy for prostate cancer, bladder cancer, and malignant glioma. Nihon Rinsho 69(9): 1657-61.

### 15. Other therapies

There are numerous other cancer therapies which can be combined with the present invention in order to create synergistic effects. Non-limiting examples are treatments targeting apoptosis, hyperthermia, hormonal therapy, telomerase therapy, insulin potentiation therapy, gene therapy and photodynamic therapy.

The present invention is further illustrated by the following examples which are not be construed as limiting the scope of the invention.

### EXAMPLES

### Example 1: Determination of mRNA expression levels by reverse transcription (RT) quantitative PCR (RT-qPCR)

RNA was isolated from paraffin-embedded, formalin-fixed tissues (= FFPE tissues). More particularly, total RNA from a 5 to 10 µm curl of FFPE tumor tissue was extracted using the High Pure RNA Paraffin Kit (Roche, Basel, Switzerland) or the XTRAKT RNA Extraction Kit XL (STRATIFYER Molecular Pathology, Cologne, Germany), quantified by the Ribogreen RNA Quantitation Assay (Molecular Probes, Eugene, OR) and/or qualified by real-time fluorescence RT-PCR of a fragment of the reference gene RPL37A or CALM2. It was recognized that differences exist between different extraction technologies when comparing quantitative data of target genes of sequential slices by different methodologies. For the purpose of the present invention the use of the XTRAKT RNA Extraction Kit XL was preferred. In general 2.5 µl RNA of each qualified extraction (approx. 50-100 ng) was assayed by qRT-PCR as described below.

For a detailed analysis of gene expression by quantitative RT-PCR methods, primers flanking the region of interest and a fluorescent labeled probe hybridizing in-between were utilized. Target specific primers and probes were selected using the NCBI prime designing tool (www.ncbi.nlm.nih.go) and/or the Primer Express® software from Applied Biosystems (Foster City, CA 94404 USA). RNA specific primer/probe sequences were used to enable RNA specific measurements by locating primer/probe sequences across exon/exon boundaries. Furthermore, primer/probes were selected not to bind to sequence regions with known polymorphisms (SNPs). In case multiple isoforms of the same gene exist, primers were selected to amplify all relevant splice variants. All primer pairs were checked for specificity by conventional PCR reactions. After further optimization of the primers/probes, the primers and probes listed in Table 1 gave the best results. To standardize the amount of sample RNA, the genes CALM2 and B2M were selected as reference genes, since they were not differentially regulated in the samples analyzed.

Name | Sequence (5' → 3') | 5' Modification | 3' Modification
MP120_For | CAGCAACTCGAGCATCATTCTT | no | no
MP120_Rev | GCAGGAGAAACTTTGCCCAAA | no | no
MP120 | TCCACCTCGACCTGCTCTCCCTGAG | FAM | TAMRA

Table 1. Used primers and probes.

TaqMan® validation experiments were performed showing that the efficiencies of the target and the control amplifications are approximately equal which is a prerequisite for the relative quantification of gene expression by the comparative ΔCT method. ΔCT values are given as maximum cycle number 40 minus ΔCT (40-(CT value target gene minus CT value housekeeping gene)). To perform the expression analysis of genes of interest within a biological sample, 4 x duplex assay-mixtures were prepared by mixing the respective primer/probes of two specific assays. For separate detection of CT values the assay probes were modified with different fluorescent probes. Each 4 x assay-mix contained 2 µM of unmodified forward and reverse primer and 1,2 µM of probe. For each reaction 2,5 µl total RNA extracted from FFPE sections (see above) was mixed with 2,5 µl assays-mix, 2,5 µl enzyme-mix and 2,5 µl water in one well of a 96-well-Optical Reaction Plate. Measurements of the PCR reaction were done according to the instructions of the manufacturer with a Versant kPCR Cycler (Siemens) or a Light Cycler 480 (Roche) under appropriate conditions (5 min. 50°C, 20 sec. 95°C, 15 sec. 95°C, 1 min. 60°C; 40 cycles). Prior to the measurement of so far unclassified biological samples control, experiments with, e.g., cell lines, healthy control samples, samples of defined molecular tumor subtypes can be used for standardization of the experimental conditions.

### Example 2: Assaying CD68 mRNA for its usefulness as a prognostic marker and for predicating cancer therapy benefit from trastuzumab treatment in cancer patients

We investigated the prognostic value of breast tumor CD68 mRNA levels within the luminal, HER2-positive and HER2 negative/ESR1 negative subtypes in the FinHer trial patient population (Joensuu H. et al. (2006) N. Engl. J. Med. 354(8):809-20), and evaluated their predictive value on survival outcomes achieved with adjuvant tratsuzumab and chemotherapy in early breast cancer.

RNA was extracted from formalin-fixed paraffin-embedded (FFPE) tumor tissue of 917 (90.8%) patients out of the 1010 patients who participated in the FinHer trial. Breast tumor CD68 mRNA content was measured using RT-qPCR from representative FFPE tissue samples. Breast cancer molecular subtypes (luminal, HER2-enriched and triple-negative) were approximated using immunohistochemistry (IHC) and central CISH testing data obtained from the FinHer trial data file. Prognostic significance of CD68 on distant disease-free survival (DDFS) was assessed using Kaplan-Meyer analysis and the log-rank test.

Within the HER2 positive breast tumors CD68 mRNA expression was normally distributed with median expression of 35.52 (40-ACT). The tumor CD68 mRNA content lower than the CD68 level of 35,18 was significantly associated with favorable DDFS in HER2-positive cancer (n=203; 90% vs. 69%; p=0.001). In the subset of HER2-positive cancer with tumor CD68 mRNA expression level higher than 35,9, patients benefitted from trastuzumab treatment (3-year DDFS 95% with trastuzumab vs. 55% without trastuzumab; p=0.016), whereas no benefit from trastuzumab was observed when tumor CD68 content was lower than 35,9 (90% vs. 88%; p=0.93); see Figures 2 and 3.

The results validate tumor CD68 concentration as a predictive biomarker in HER2-positive early breast cancer. Patients with HER2-positive cancer and with high tumor CD68 mRNA content (indicating low tumor macrophage content) benefitted from trastuzumab treatment, whereas patients with HER2-positive breast cancer with low tumor CD68 mRNA content (indicating high tumor macrophage content) had no benefit from adjuvant trastuzumab. Trastuzumab may be effective only for macrophage-rich HER2-positive cancers that are prone to antibody-dependent cellular cytotoxicity (ADCC), whereas it may have little efficacy for cancers that progress due to low intratumoral macrophage content. Other agents, such as T-DM1, might work better than trastuzumab in the subset of women who have HER2-positive BC with a high tumor macrophage content.

### Example 3: Assaying CD68 mRNA for its usefulness as a marker for predicting cancer therapy benefit from chemotherapy

Pretreatment core cut biopsies from n=100 patients with PBC (primary breast cancer) treated within a randomized phase II trial of anthracyline/taxane based NAC (neoadjuvant chemotherapy) were examined (Schneeweiss et al. (2011) Ann. Oncol. 22(3):609-17). RNA from formalin-fixed, paraffin embedded (FFPE) routine biopsies were extracted using a bead-based extraction method (STRATIFYER XTRAKT kits). CD68 and ESR1, PGR, HER2, Ki67 as well as CALM2 as a house keeping gene were measured via a multiplex quantitative RT-PCR (RT-qPCR). Correlation analyses and partitioning tests were performed using the SAS JMP® 9.0.0 software.

CD68 mRNA exhibited a normal data distribution (Median expression 40-DCT 34,66) in the core needle biopsies of advanced breast tumors. CD68 mRNA correlated strongly with Ki67 mRNA levels (Spearman r=0,46; p<0,001), whereas there is virtually no correlation with other classical biomarkers such as ESR1, PGR and HER2. Partitioning analysis revealed that CD68 mRNA levels are superior to the previously and prospectively validated response markers ESR1 mRNA and HER2 mRNA. When using the median mRNA expression as an objective cut-off measurement no tumor having low CD68 mRNA expression did respond to chemotherapy, while 22% of the CD68 positive tumors responded by pathological complete response. Based on molecular subtyping into luminal, HER2 positive and HER2 negative/ESR1 negative with predefined Cut-Off values for ESR1 mRNA and HER2 mRNA levels, high CD68 were particularly informative within luminal tumors (22% pCR versus 0 % pCR rate) and HER2 negative/ESR1 negative tumors (38% pCR versus 0% pCR rate).

The presence of high levels of CD68 (indicating the presence of high levels of macrophages) indicates tumors being sensitive to chemotherapy beyond the prospectively validated ESR1 and HER2 mRNA determinations, both in luminal and HER2 negative/ESR1 negative tumors. The mRNA expression levels of CD68 provide additional information beyond conventional subtyping and might therefore be useful for early assessment of non-response to chemotherapy und subsequent treatment planning.

## Claims

1. A method of assessing if a cancer patient responds to or benefits from treatment with an antibody against an antigen on tumor cells, acting through recruiting the patient's immune system to destroy tumor cells, said method comprising determining the level of cells of the macrophage lineage in a tumor sample obtained from the patient.

2. The method of claim 1 wherein the presence of cells of the macrophage lineage or a level of cells of the macrophage lineage which is lower than a defined threshold indicates an increased risk of a cancer patient not responding to or benefiting from treatment with the antibody.

3. The method of claim 1 or 2 wherein the absence of cells of the macrophage lineage or a level of cells of the macrophage lineage which is higher than a defined threshold indicates a reduced risk of a cancer patient not responding to or benefiting from treatment with the antibody.

4. The method of any one of claims 1 to 3 wherein the level of cells of the macrophage lineage is determined by determining the expression level of one or more macrophage specific markers, wherein one or more macrophage specific markers are preferably selected from the group consisting of: CD68, CD14, CD163, CD80, MAC2 (galectin-3), EMR1-F4/80, CD11b and CD205.

5. A method of assessing if a cancer patient having a HER2-positive tumor is a responder to treatment with an antibody against the HER2 protein acting through recruiting the patient's immune system to destroy tumor cells, said method comprising determining the expression level of CD68 in a tumor sample obtained from the patient.

6. The method of claim 5 wherein expression of CD68 or an expression level of CD68 which is lower than a defined expression threshold indicates an increased risk of a cancer patient not being a responder to treatment with the antibody or not benefiting from treatment with the antibody.

7. The method of claim 5 or 6 wherein no expression of CD68 or an expression level of CD68 which is higher than a defined expression threshold indicates a reduced risk of a cancer patient not being a responder to treatment with the antibody or not benefiting from treatment with the antibody.

8. The method of any one of claims 1 to 7 wherein the antibody acts through antibody-dependent cell-mediated cytotoxicity (ADCC) and/or complement-dependent cytotoxicity (CDC).

9. A kit comprising means such as reagents for determining the level of cells of the macrophage lineage in a sample isolated from a patient.

10. A method of treating a cancer patient, said method comprising
a. assessing if the cancer patient is a responder to treatment with an antibody acting through recruiting the patient's immune system to destroy tumor cells by the method of any one of claims 1 to 8 and
b. (i) treating the cancer patient with an antibody acting through recruiting the patient's immune system to destroy tumor cells if the patient has a reduced risk for not being a responder to treatment with the antibody or (ii) treating the cancer patient with a treatment regimen which comprises a treatment which is different from a treatment with an antibody acting through recruiting the patient's immune system to destroy tumor cells if the patient has an increased risk for not being a responder to treatment with the antibody, wherein said treatment regimen preferably does not comprise a treatment with an antibody acting through recruiting the patient's immune system to destroy tumor cells.

11. A method of assessing if a cancer patient is a responder to treatment with chemotherapy, said method comprising determining the level of cells of the macrophage lineage in a tumor sample obtained from the patient.

12. A method of assessing if a cancer patient is a responder to treatment with chemotherapy, said method comprising determining the expression level of CD68 in a tumor sample obtained from the patient.

13. A method of treating a cancer patient, said method comprising
a. assessing if the cancer patient is a responder to treatment with chemotherapy by the method of any one of claims 11 or 12 and
b. (i) treating the cancer patient with chemotherapy if the patient has a reduced risk for not being a responder to treatment with chemotherapy or (ii) treating the cancer patient with a treatment regimen which comprises a treatment which is different from chemotherapy if the patient has an increased risk for not being a responder to treatment with chemotherapy, wherein said treatment regimen preferably does not comprise chemotherapy.

14. A method of assessing the clinical outcome for a cancer patient, said method comprising determining the level of cells of the macrophage lineage in a tumor sample obtained from the patient.

15. A method of assessing the clinical outcome for a cancer patient, said method comprising determining the expression level of CD68 in a tumor sample obtained from the patient.
